(19) Europäisches Patentamt
European Patent Office
Office européen des brevets

(11) Publication number: **0 411 135 A1**

(12) **EUROPEAN PATENT APPLICATION published in accordance with Art. 158(3) EPC**

(21) Application number: 90901009.2

(22) Date of filing: 19.12.89

(86) International application number: PCT/JP89/01275

(87) International publication number: WO 90/06998 (28.06.90 90/15)

(51) Int. Cl.5: C12N 15/13, C12Q 1/68

(30) Priority: 19.12.88 JP 319809/88

(43) Date of publication of application: 06.02.91 Bulletin 91/06

(84) Designated Contracting States: DE FR GB IT

(71) Applicant: MITSUI TOATSU CHEMICALS, Inc.
2-5 Kasumigaseki 3-chome
Chiyoda-Ku Tokyo 100(JP)

Applicant: EDUCATIONAL FOUNDATION FUJITA GAKUEN
12-1, Minamiyakata, Sakaemachi
Toyoake-shi, Aichi 470-11(JP)

(72) Inventor: KUROSAWA, Yoshikazu 1-39, Ohgimachi Meitoh-ku
Nagoya-shi
Aichi 465(JP)
Inventor: ICHIHARA, Yoshikazu Nakajima Haitsu 101
17-7, Futamuradai 4-chome Toyoake-shi
Aichi 470-11(JP)
Inventor: AWAYA, Akira Yabecho Daini Apaato 1541, Yabecho
Totsuka-ku Yokohama-shi
Kanagawa 244(JP)
Inventor: ISHIZUKA, Yusaku 21, Honmokuoosatocho Naka-ku
Yokohama-shi
Kanagawa 231(JP)

(74) Representative: Holdcroft, James Gerald, Dr. et al
Graham Watt & Co., Riverheadead
Sevenoaks, Kent TN13 2BN(GB)

(54) **DNA FRAGMENTS RELATED TO HUMAN IMMUNOGLOBULIN GENES AND METHOD OF DIAGNOSIS USING SAID FRAGMENTS.**

(57) The present of $D_H$ genes belonging to any of the $D_H$ gene families $D_A$,$D_K$ and $D_{IR}$ has been proved and DNA fragments containing at least one of these $D_H$ genes have been cloned. The DNA fragments are useful as a probe in the screening and identification of various $D_H$ genes and as a gene arrangement to be used in various genetic engineering techniques for the artificial preparation of immunoglobulin shaving various amino acids at specified sites. Furthermore the use of these DNA fragments makes it possible to detect and analyze rejoining parts of $V_HD_HJ_H$ and utilize DNA fragments selected on the basis of the DNA sequences thus clarified as the tumor marker, thereby allowing the detection of tumor cells of the B-cell or T-cell type, or the diagnosis of lymphoid tumor, leukemia, etc.

EP 0 411 135 A1

# DNA FRAGMENTS RELATED TO IMMUNOGLOBULIN GENE AND METHOD OF DIAGNOSIS USING SAID DNA FRAGMENTS

## TECHNICAL FIELD

The present invention concerns new DNA fragments which relate to the production of immunoglobulins or T-cell receptors in animals ranging from lower animals and higher animals such as mammalians, particularly in humans, which produce immunoglobulins or T-cell receptors, in the fields of immunology, genetics, embryology, hematology and cancer research.

By analyzing and demonstrating, at the gene level, diverse reactivities of the DNA fragments of the present invention with immunoglobulin or T-cell receptors, by cloning from different cells and determining structures, necessary information can be provided to establish techniques to produce various immunoglobulins having various antigenic specificities using expression process of the genes contained in these DNA fragments.

Furthermore, the present invention also provides a method of detecting B-cell or T-cell line tumor cells using characteristic DNA fragments containing the above-mentioned specified DNA fragments, namely a method of diagnosing lymphoid neoplasm, leukemia or the like.

## BACKGROUND ART

Owing to the development in immunological chemistry, cellular immunology and immunological genetics, the following facts have been elucidated in regard to antibody molecules (immunoglobulins) produced in animals (see Reference 1: details are described in the list of references described later).

An immunoglobulin (Ig) molecule consists of two each of identical heavy (H) chain and light (L) chain. Each H and L chain is structurally and functionally divided into two regions: a region called the constant (C) region which is located in the C-terminal site and has an invariant primary structure and a region called the variable (V) region, consisting of about 110 amino acid residues, which is located in the C terminal site; the V regions correspond to antigen binding sites.

The multiplicity of antigen binding capability of antibodies is explained by the differences in primary amino acid sequences in V regions; namely, the multiplicity is attributed to the differences in the DNA nucleotide sequences in the corresponding genes.

The multiplicity of the amino acid sequences in V region of the H and L chains is generally great through the whole area of V regions; however, portions with particularly great multiplicity are concentrated in three sites of each chain. These three sites are located between polypeptides II and III, III and IV, and VI and VII, the polypeptide forming the beta-sheet configuration in the three-dimensional construction of the V regions in Ig; thus, the sites in H and L chains total six. These sites are called the complemetarity determining regions (CDR) because they form antigen binding sites in the pockets.

Further, these these sites are individually called CDRI, CDRII and CDRIII.

Among them, CDRIII in H chains is particularly diversed, which closely associated with the fact that the site of this V region has the capability to bind different antigens.

Among the 20 kinds of amino acids, ten amino acids that can form the beta-sheet configuration with relative ease are cystine, phenylalanine, isoleucine, leucine, methionine, glutamine, threonine, valine, tryptophan, tyrosine.

On the other hand, the total organization of the genes of Ig H chain in order is as follows: H chain V (variable) region (hereinafter referred to as $V_H$) genes, H chain D (diversity) region (hereinafter referred to as $D_H$) genes, H chain J (joining) region (hereinafter referred to as $J_H$) genes and C region genes.

Ig genes are those expressed specifically in B-lymphocyte-related cells and the expression is controlled in accordance with the process of differentiation and maturation of B cells.

Namely, these genes undergo two kinds of DNA rearrangements; one is a $V_H$-$D_H$-$J_H$ joining, resulting in the formation of a completely active- type V gene. The other is DNA recombination for the H chain class switch.

H chain genes scattered throughout the embryonic-type (germline) gene sequence are transposed and assembled as follows: In the process of differentiation from stem cells to B cells, first, one $D_H$ gene fragment and one $J_H$ gene fragment are bound whereas intron portions containing signal heptamers, signal nonamers and spacers are deleted; then, this D-J joining fragment is bound to one $V_H$ fragment to form an active-type V gene having a V-D-J gene sequence.

The $D_H$ gene present in the D region of this Ig H chain gene is a gene coding for CDRIII of the H chain; the embryonic-type $D_H$ gene which belongs to the same family is repeatedly encoded at regular intervals: 5 kb in mice (Reference 2) and 9 kb in humans (Reference 3).

However, regarding the distribution and mode existence of $D_H$ genes in the D regions, detailed investigation has not so far been carried out sufficiently, and only two $D_H$ gene families ($D_{HQ52}$ and $D_{LR}$) have been identified as shown in the above-mentioned report by Siebenlist et al. (Reference 3).

Accordingly, one of the present inventors identified 12 mouse embryonic-type $D_H$ genes and classified them into three families (Reference 2); thereafter, the present inventors investigated human $D_H$ region genome in detail and identified five different $D_H$ genes ($D_{M1}$ gene, $D_{LR1}$ gene, $D_{XP1}$ gene, $D_{XP'1}$ gene and $D_{N1}$ gene) in a 9 kb unit (Reference 4).

Along with the $V_H$ gene regions and $J_H$ gene regions, the $D_H$ gene regions determine different reactivities of the antibody molecules responding to different antigens; it is said that one organism can produce $10^6$ to $10^8$ kinds of antibody molecules using the combination of the bindings of these three genes.

On the other hand, diverse combination of V-D-J joinings of genes of T-cell receptors, particularly the beta-chain, and delta-chain and diverse reactivity responding to antigens have been elucidated analogously to immunoglobulins.

Thus, the outline of the mechanism, in which B-cell related cells or T-cell related cells are differentiated responding to various antigens and rearrangements occur between individual genes of antibodies or T-cell receptors during the differentiation process to produce various antigens or T-cell receptors, has been elucidated; however, many problems have remained unsolved to verify the mechanism, and research on artificial production of various antibodies or the like by using the mechanism has not yet been carried out.

## DISCLOSURE OF THE INVENTION

The present inventors attempted to reveal the entire $D_H$ gene regions which are closely associated particularly with determination of multiplicity of the antigen binding capability of CDRIII so as to open the way to develop means to create a variety of antibodies.

Namely, the present inventors planned to clone further more $D_H$ genes and reveal the nucleotide sequence of these $D_H$ genes to use these $D_H$ genes for artificial preparation of Ig having various altered amino acids in specified sites.

In conventional research on antibody production, in most cases, polyclonal antibodies and monoclonal antibodies responding to particular antigens are prepared under the necessity; however, in the method by the present inventors, in contrast to the conventional methods, different antibodies are first artificially prepared and then the antibodies against antigens which are not yet investigated or newly found are searched and screened among them.

An object of the present invention is to provide extremely useful new technology for elucidation of the regulatory mechanism of production of Ig and its applications useful in artificial production of Ig in the field of cell biology, medical science or medicinal industry.

For example, an object of the present invention is to provide detailed sequences of various $D_H$ genes, and probes useful for screening and identification of the various $D_H$ genes.

Another object of the present invention is to provide a method of detecting B-cell related or T-cell related tumor cells using characteristic DNA fragments containing DNA fragments of the various $D_H$ genes described above as tumor markers, namely to provide a method of diagnosting lymphoid neoplasm, leukemia or the like.

The DNA fragments of the present invention are characterized in that they contain at least one or more $D_H$ genes belonging to $D_H$ gene family $D_A$, $D_K$ or DIR.

These DNA fragments are useful as probes for screening and identification of various $D_H$ genes or as gene sequences used for various genetic engineering manipulation in order to artificially prepare Ig in which amino acids at specific sites are altered variously.

Furthermore, $V_H$-$D_H$-$J_H$ rejoining sites are detected and analyzed using the DNA fragments of the present invention, and the DNA fragments selected based on the DNA sequences thus obtained are used as tumor markers for detection of B-cell or T-cell-related tumor cells; thereby diagnosis of lymphoid neoplasm, leukemia or the like becomes possible.

In conventional research and practice regarding antibody production, antibodies responding specified antigens are produced as polyclonal antibodies in animals or as monoclonal antibodies in mouse or human antibody-producing hybridomas.

It is possible to produce at least 90 kinds of antibodies, only for a $D_H$ region, using 30 kinds of $D_H$

genes obtained according to the present invention or three different combinations of open reading frames. By combining $V_H$ regions and $J_H$ regions, an enormous kinds of antibodies can be produced; however, on the basis of the knowledge obtained according to the present invention, a new method is conceived, in which various antibodies are prepared first by methods in cellular engineering and genetic engineering and thereafter desired antibodies responding to antigens that is not so far fully investigated or newly found are searched and screened among them. If this method is generalized, it is possible to provide, at needs, specified antibodies which are urgently needed and in a large quantity to be used for medicinal or research purposes.

Furthermore, according to the present invention, it is revealed that DNA sequences of the $V_H$-$D_H$-$J_H$ rearranged joining sites containing various $D_H$ genes have almost unlimited diversity and that $D_H$ genes also identified according to the present invention are contained in fact in $D_H$ regions of tumor cells of patients. Furthermore, based on these observations and on the fact that tumor cells of one tumor patient are monoclonal so that V (N-D-N) J region in the tumor cells of the individual patient has a characteristic DNA sequence, a DNA fragment marker characteristic to the patient is synthesized, using the DNA sequence as a characteristic, for the use as a primer in multiplication by the PCR method; the DNA sequences characteristic to the tumor cells of the patient is amply produced and the percentage of the tumor cells to other lymphatic cells can be evidently calculated and thus a diagnostic method of the present invention has been established.

This diagnostic method is far sensitive as compared with conventional morphological or cellular genetical diagnostic method and thus considered to be an accurate testing method, which is useful for early diagnosis and evaluation of chemotherapy in leukemia, lymphoid neoplasm or the like.

Furthermore, various $D_H$ probes obtained according to the present invention are useful as a diagnostic medicine to be used in cellular typing of lymphocytes of various patients:

Furthermore, immunoglobulin genes and related genes can be more easily prepared by using the $D_H$ probes according to the present invention.

## BRIEF DESCRIPTION OF DRAWINGS

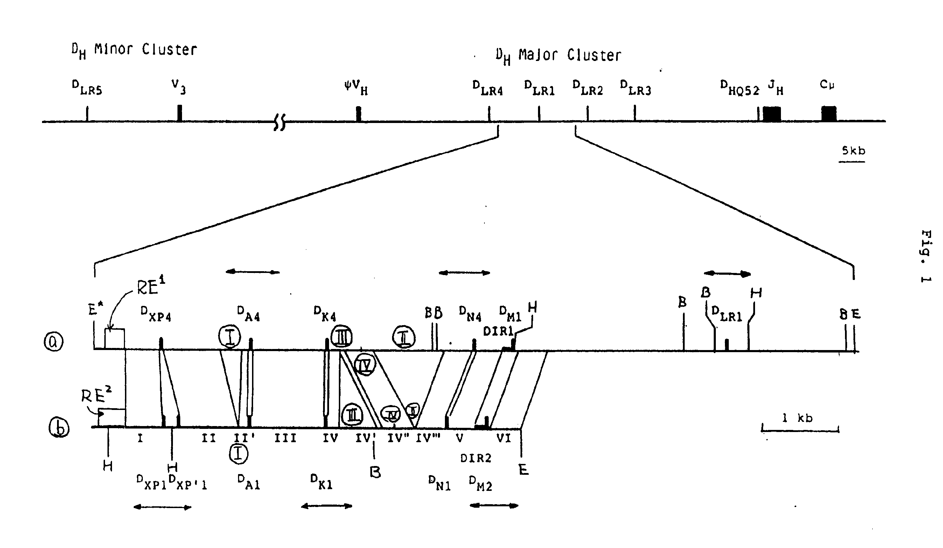

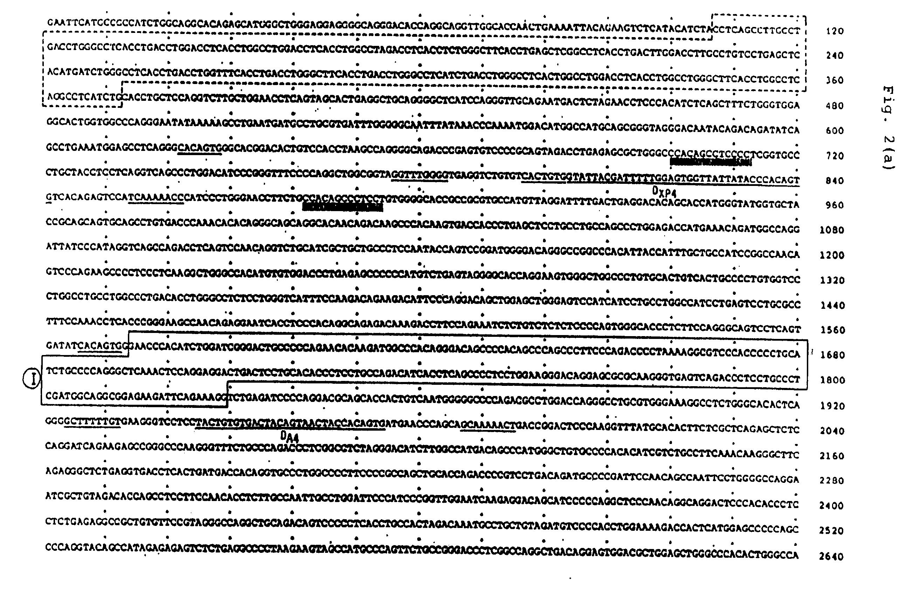

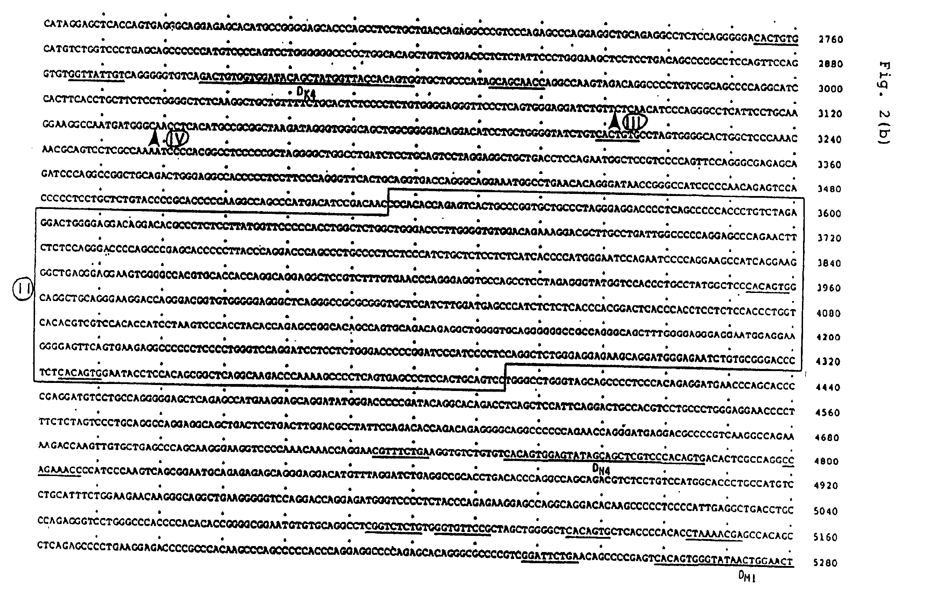

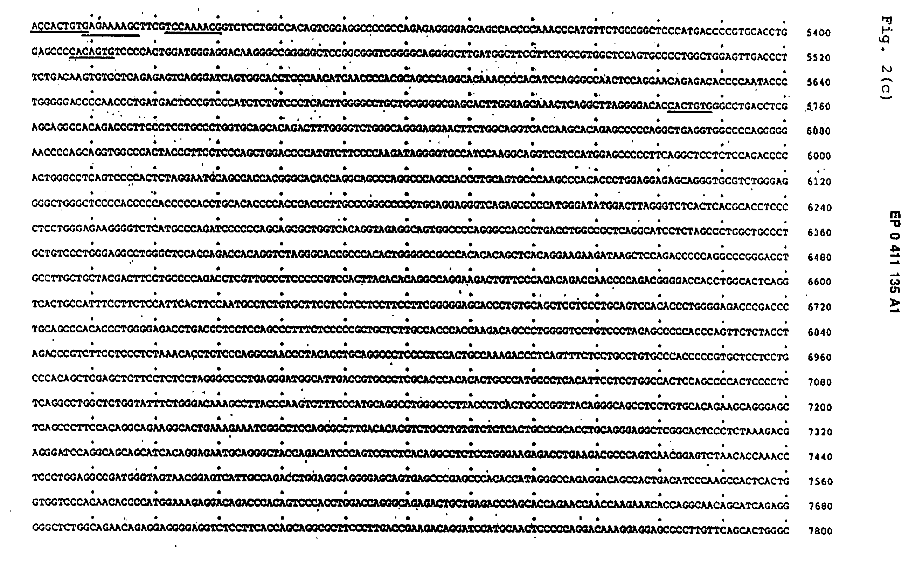

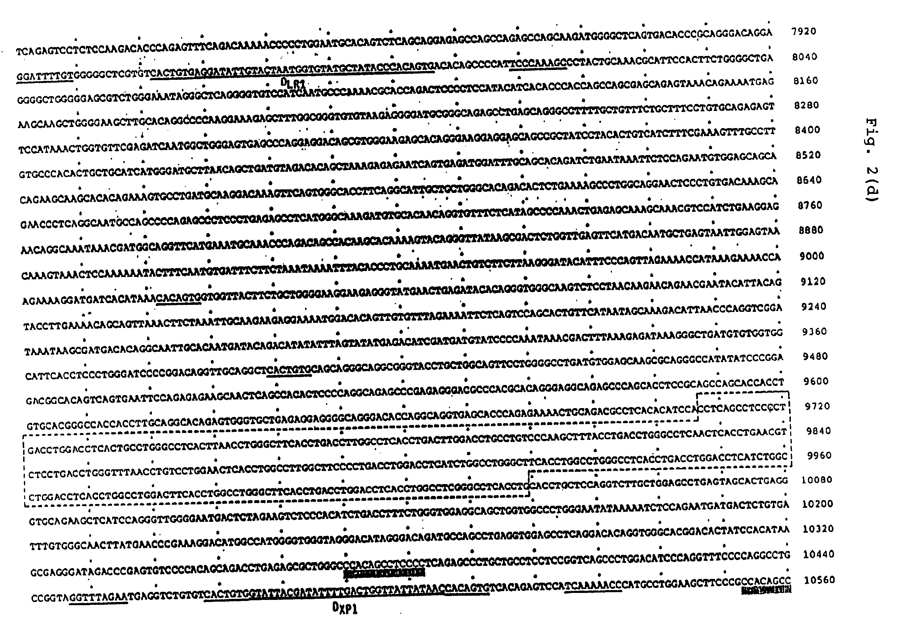

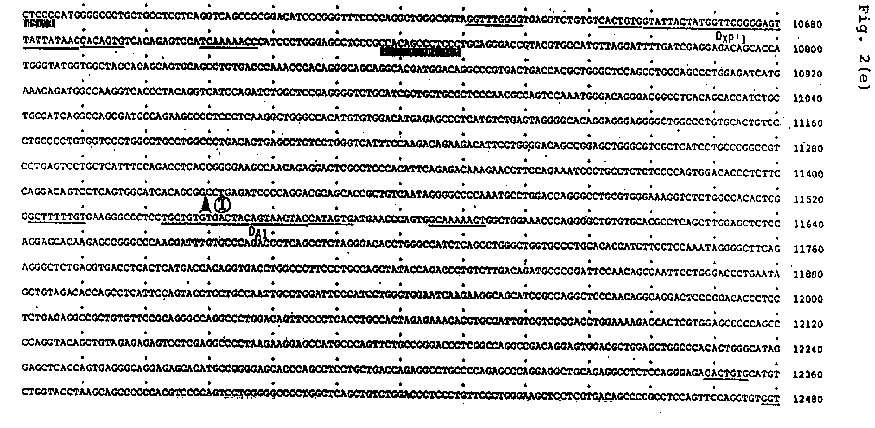

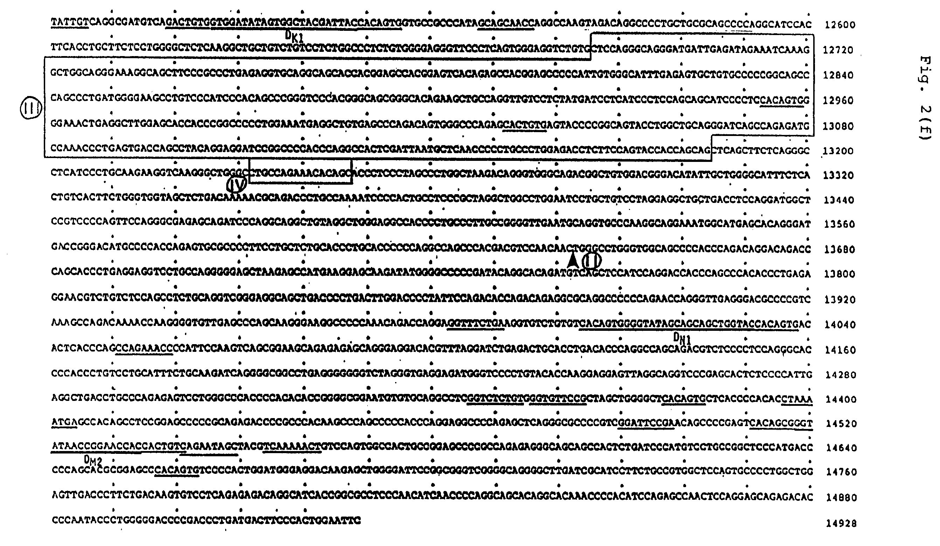

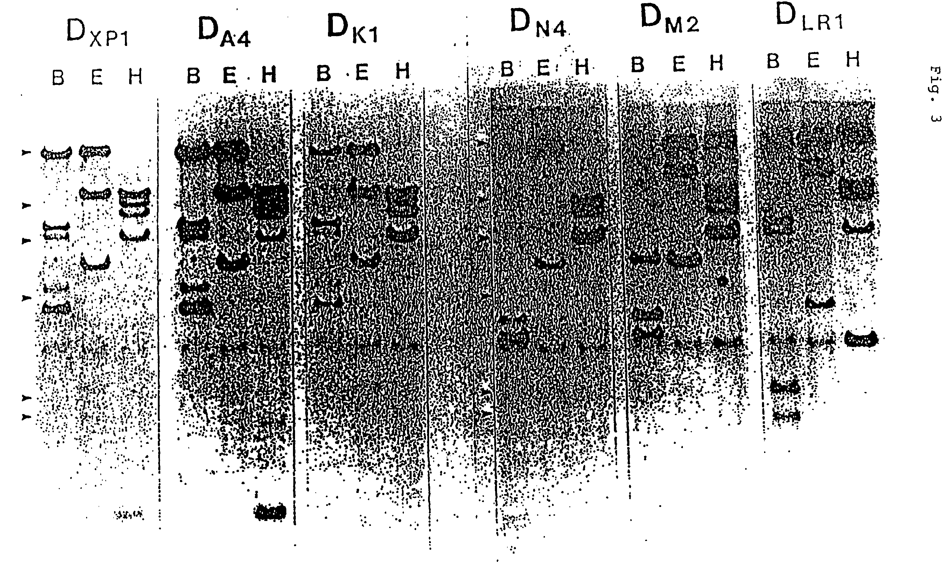

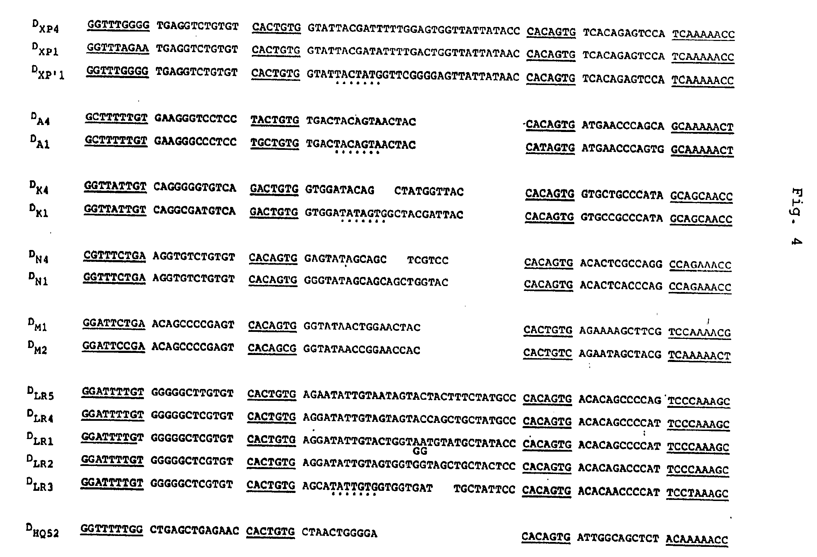

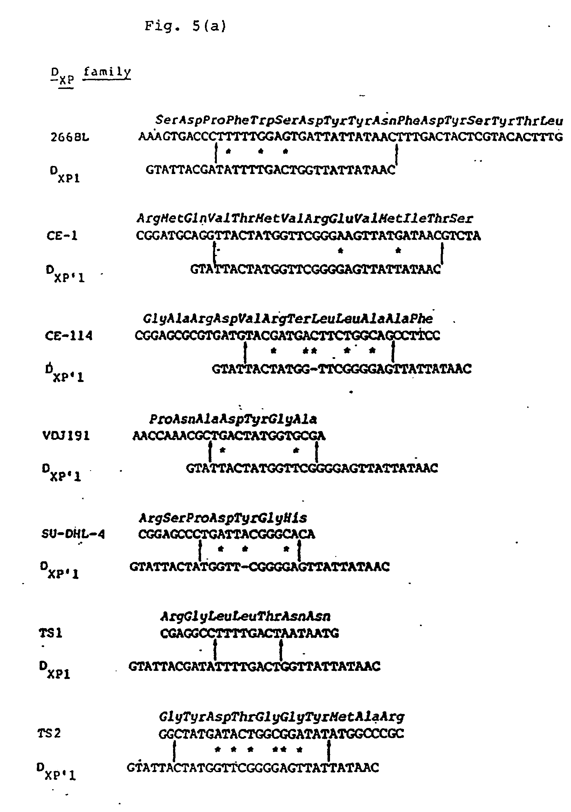

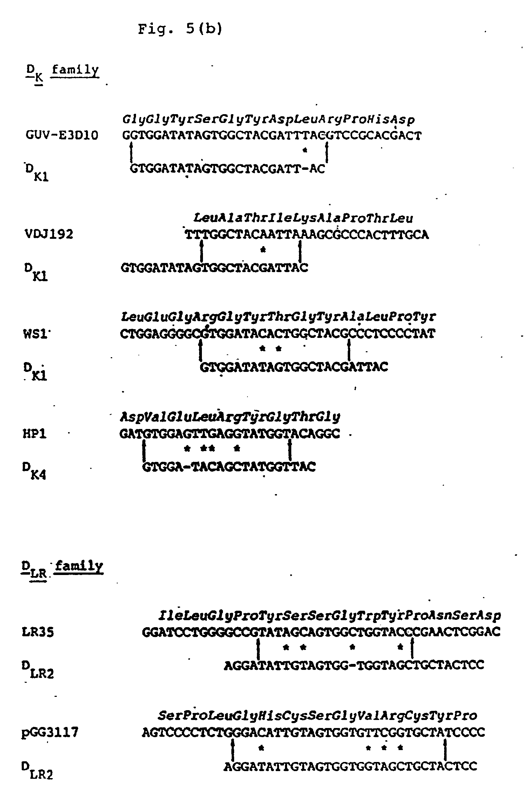

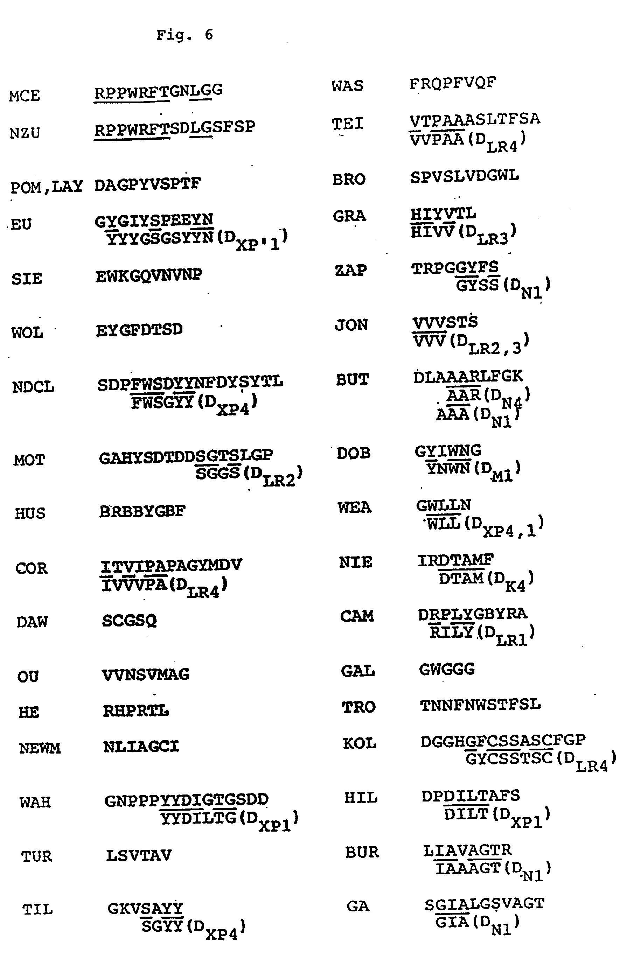

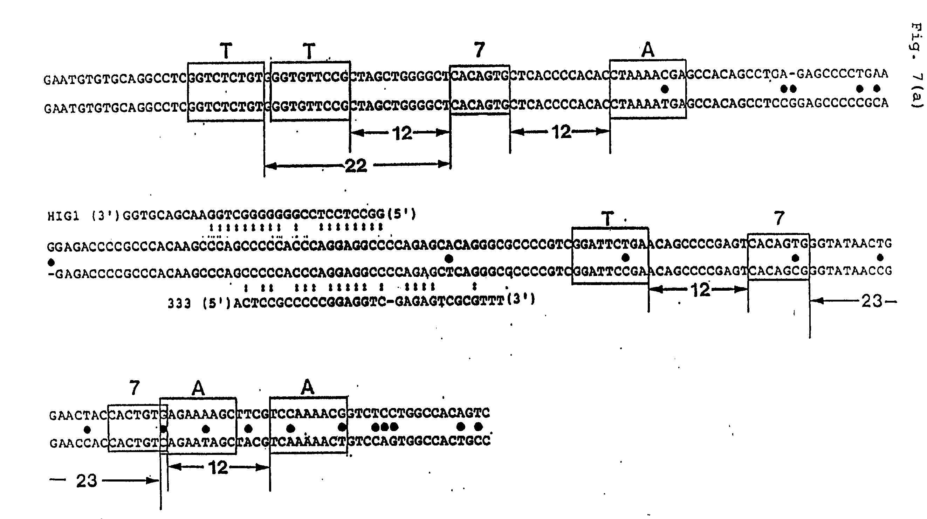

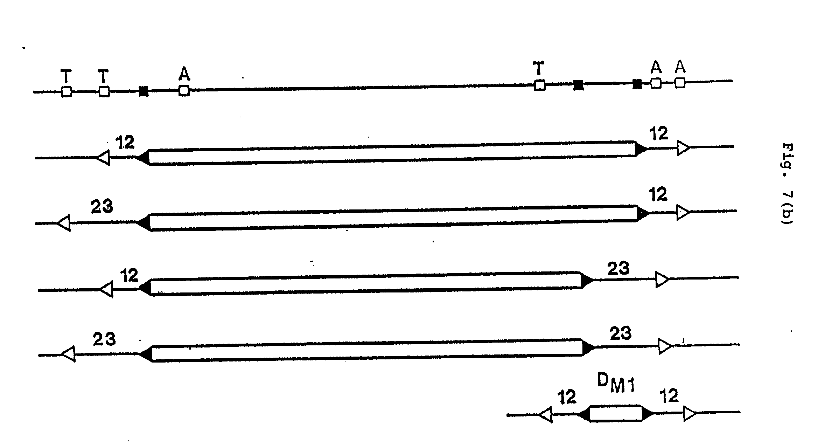

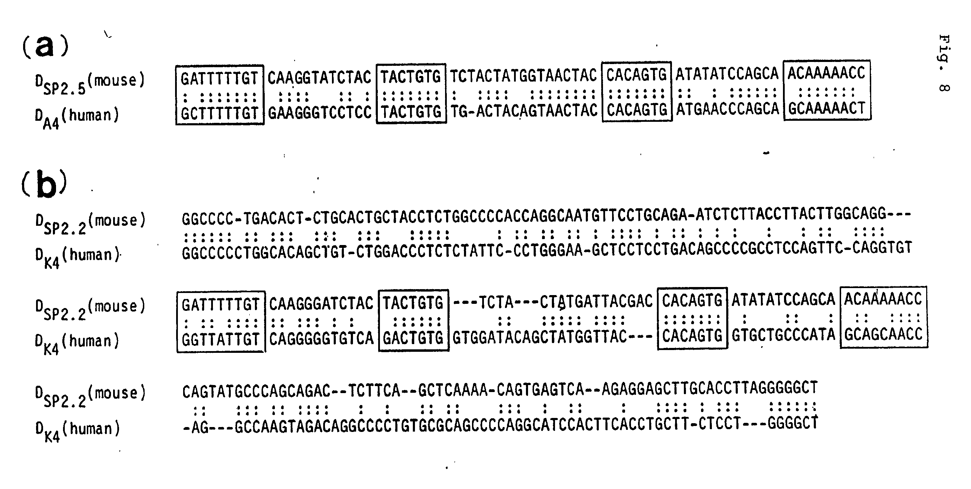

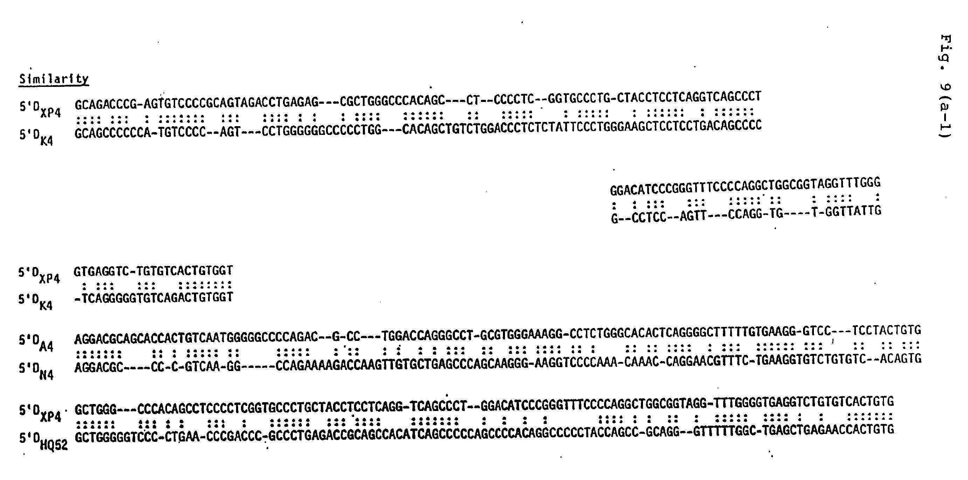

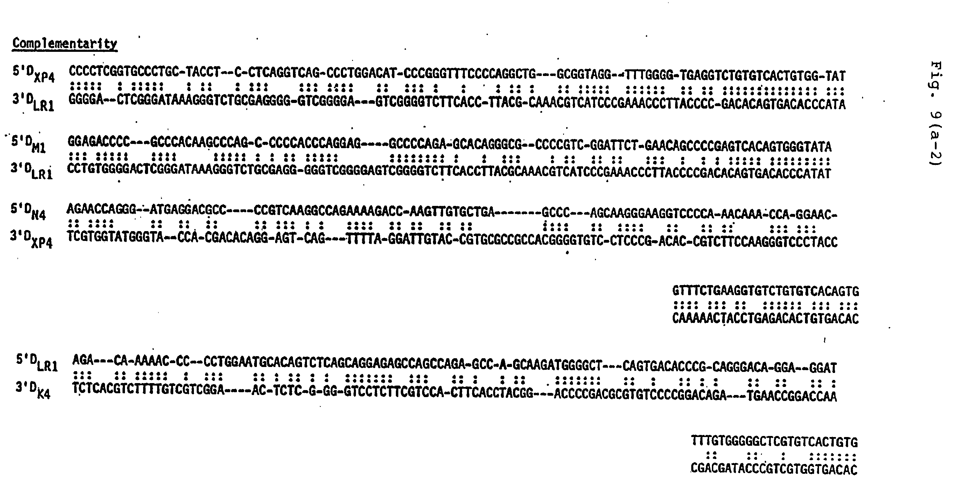

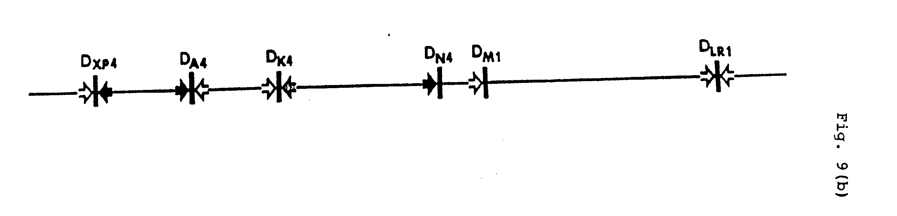

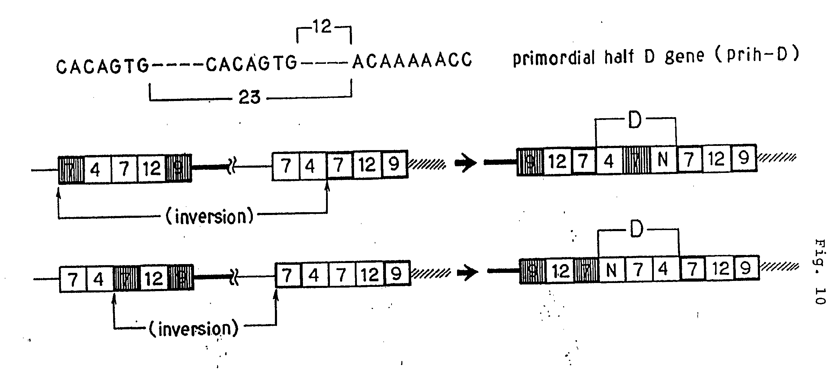

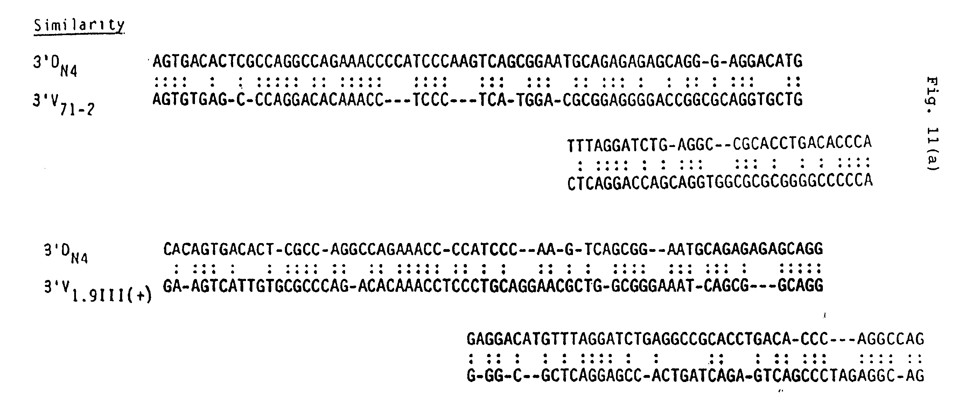

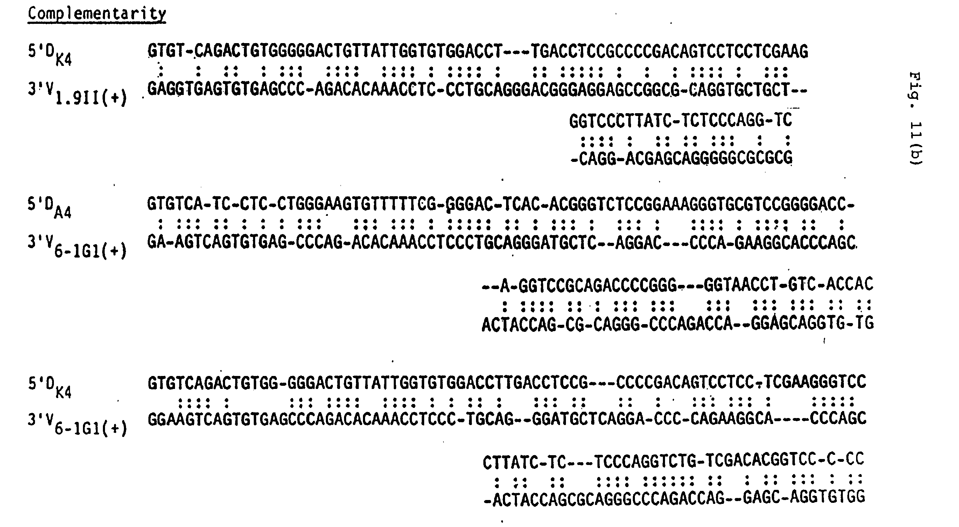

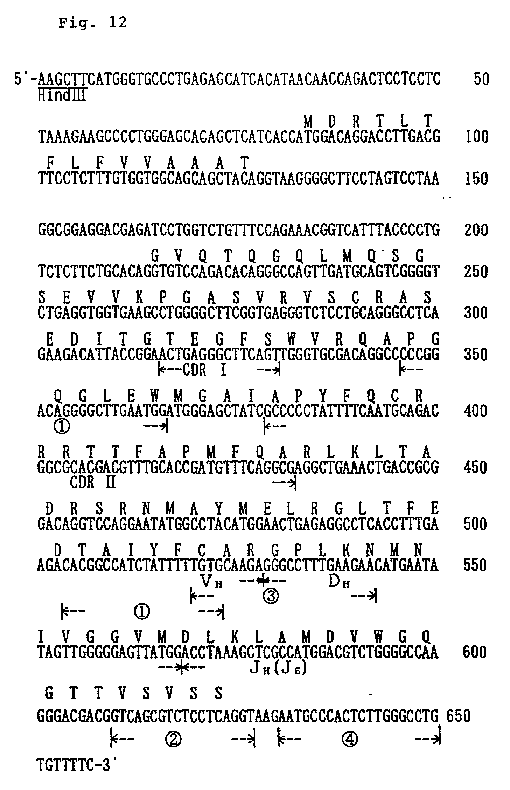

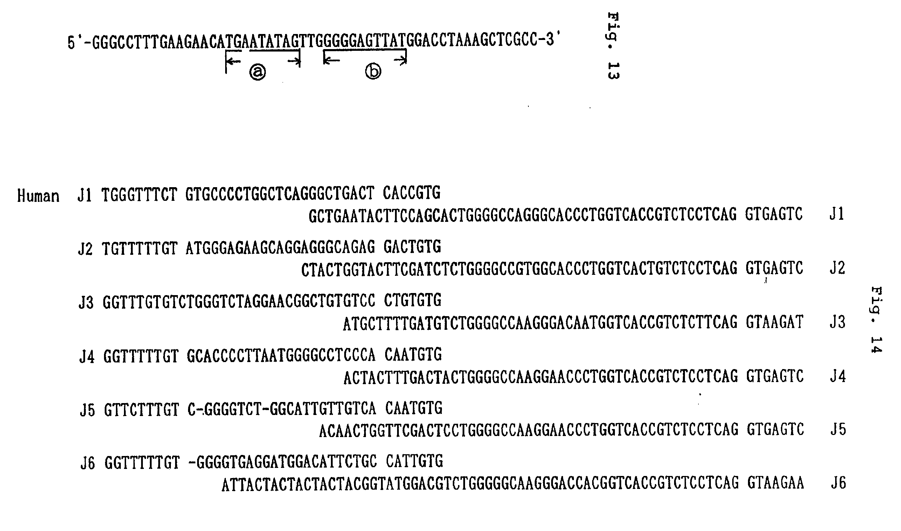

Figure 1 is a schematic diagram showing locations of the sequences of 12 $D_H$ genes in the 15 kb DNA fragment according to the present invention; Figures 2(a) to 2(f) illustrate the nucleotide sequence of the 15 kb DNA fragment containing 12 $D_H$ genes shown in Figure 1; Figure 3 illustrates the results of the Southern hybridization carried out in Example 3; Figure 4 shows the nucleotide sequence of 17 human embryonic-type genes containing $D_H$ genes found in the 15 kb DNA fragment shown in Figure 1; Figures 5(a) and (b) are shown to demonstrate comparison of nucleotide sequences between embryonic cell-type $D_H$ genes and somatic-cell-type $D_H$ genes; Figure 6 is shown to demonstrate comparison, at the level of amino acid sequences, of somatic-cell-type $D_H$ with those of embryonic-cell-type $D_H$; Figures 7 (a) and (b) show a construction of DIR gene family; Figures 8 (a) and (b) are shown to demonstrate comparison between mouse $D_H$ sequences and human $D_H$ sequences; Figures 9(a-1), (a-2) and (b) are shown to demonstrate comparison of nucleotide sequences between sequences adjoining in various $D_H$ genes; Figure 10 is a schematic diagram showing a model of the birth of $D_H$ genes (the figures in squares indicate numbers of nucleotides); Figures 11 (a) and (b) are shown to demonstrate comparison of the sequences between regions of both adjacent ends of $D_H$ genes and the adjacent region of the 3' end of embryonic-type $V_H$ genes; Figure 12 shows the DNA sequence of the $V_H$-$D_H$-$J_H$ region derived from a IgD-secreting myeloma patient (circled 1 to 4 represent sequences used for selection of primers, and alphabets given on the nucleotide sequences represent amino acids corresponding to the codons; amino acids are identified as shown in Figure 6); Figure 13 shows the DNA sequence of the $D_H$ region only, shown in Figure 12 (circled a represents the sequence corresponding to $D_{K1}$ and circled b represents the sequence corresponding to $D_{XP'1}$); and Figure 14 shows a part of results of identification of various $J_H$ regions.

## BEST MODE FOR CARRYING OUT THE INVENTION

The DNA fragments according to the present invention are characterized in that they contain at least one or more $D_H$ genes belonging to $D_H$ gene family $D_A$, $D_K$ or DIR.

The DNA fragments according to the present invention will be hereinafter explained in detail using a DNA fragment consisting of 15 kb as a representative example, which contains $D_{XP4}$ gene, $D_{A4}$ gene, $D_{K4}$ gene, $D_{N4}$ gene, $DIR_1$ gene, $D_{M1}$ gene, $D_{LR1}$ gene, $D_{XP1}$ gene, $D_{XP'1}$ gene, $D_{A1}$ gene, $D_{K1}$ gene, $D_{N1}$ gene,

$DIR_2$ and $D_{M2}$ gene.

Further, the present inventors first specified the existence and substantial DNA sequences of $D_H$ genes found in the 15 kb DNA fragment, belonging to gene families $D_A$, $D_K$ and DIR, $D_{A4}$ gene and $D_{A1}$ gene belong to $D_A$, $D_{K4}$ gene and $D_{K1}$ gene belonging to $D_K$ and $DIR_1$ and $DIR_2$ belonging to DIR.

As mentioned above, it is known that $D_H$ genes belonging to the same family are repeated at 9 kb intervals in the $D_H$ gene region (a region where a large number of $D_H$ genes are sequenced), and $D_H$ genes, which belong to the same family as the above-mentioned $D_H$ genes identified by the present inventors, exist also in $D_H$ gene regions besides said 15 kb DNA fragment region (this was supported by the results obtained in Examples hereinafter).

Consequently, $D_H$ genes which belong to gene family $D_A$, $D_K$ or $D_{IR}$ and exist in $D_H$ gene regions besides the above-mentioned 15kbp DNA fragment region are also included in the present invention.

Figure 1 is a schematic diagram showing the location of said 15 kb DNA fragment region in human embryonic type DNA.

Said 15kb DNA fragment region corresponds to upstream and downstream portions of $D_{LR1}$ equivalent to $D_1$ described in the above-mentioned report (Reference 3) by Siebenlist et al.; the upstream and downstream portion containing $D_{LR1}$ consisting of 9kb are designated as the circled a and b, respectively, and shown separately so as to correlate $D_H$ genes of the same families. Namely, the 5' terminal of the DNA chain marked with the circled b is linked to the 3' terminal of the DNA chain marked with the circled a.

Lines drawn between the DNA chain with circled a and the DNA chain with circled b show the sites of insertion or deletion in comparison of the two chains.

Further, the regions marked with $RE^1$ and $RE^2$ represent repeats of 16 bp. Furthermore, E, B and H represent restriction cleavage sites for EcoRI, BamHI and HindIII, respectively. $E^*$ is an EcoRI cleavage site which is introduced by Maniatis et al. for artificial cloning.

Accordingly, in order to obtain a small fragment containing a desired portion from said 15kb DNA fragment, these restriction enzyme cleavage sites can be preferably used.

The above-mentioned 15 kb DNA fragment and each $D_H$ gene according to the present invention are useful as materials in utilizing functions of these DNA fragments for various purposes, for example in preparation of various artificial antibodies using genetic engineering, and as agents to be used in order to confirm new findings by the inventors as explained hereinafter or to further elucidate mechanisms to control immunoglobulin production.

The 15kb DNA fragment and each $D_H$ gene according to the present invention can be prepared by a method comprising treating, for example, human immunoglobulin heavy chain genes with appropriate restriction enzymes to make a fragments, ligating these fragments to appropriate vectors, constructing a DNA library and then selecting clones containing desired genes from the library. Furthermore, $D_H$ genes can be prepared by re-cloning from the 15kb DNA fragment or by chemical synthesis.

For construction of the DNA library, vectors comprising various plasmids and phages extensively used for cloning can be used.

An example in which a vector derived from lambda phage is used as a vector will be described below.

First, an appropriate haematopoietic cell DNA containing human immunoglobulin heavy chain DNA genes is prepared according to the ordinary method and then treated with appropriate restriction enzymes, and the resultant DNA fragments are ligated into a vector derived from lambda phage such as Charon 4A vector so as to construct a DNA phage library.

Subsequently, from this DNA phage library, appropriate recombinants which hybridize with probes for $D_H$ gene screening are selected according to the plaque hybridization method or the like.

Whether the selected clones contain desired genes can be confirmed by constructing restriction maps or analyzing DNA sequences.

Further, an example of the above-mentioned DNA phage library to be used is Maniatis' human DNA phage library (Reference 5).

Further, this screening is carried out, for example, by the Benton-Davis method (Reference 6).

Further, the clones with insertion of desired genes into vectors derived from lambda phage are multiplied in suitable bacterial cells as a host so as to make them applicable for various usages.

As in the case of Maniatis' human DNA phage library where Charon 4A vector is used, Escherichia coli DP 50, E. coli 803 [for example, provided by K. Murray (University of Edinburgh)], E. coli K-12 x 1776 (ATCC 31244;), E. coli K 802 (ATCC 33526), E . coli LE 392 (ATCC 33572), E. coli MM 29d (ATCC 33625) or E. coli MM21 (ATCC 33678) can be used as a host. Among these strains, E. coli 803 is preferably used.

Clones containing the above-mentioned 15kb DNA fragment can be isolated, for example, by the above-mentioned Benton-David method (Reference 6) using the $D_H$-$J_H$ probe isolated from the above-mentioned Maniatis's human DNA phage library by Ravetch et al. (Reference 10).

Further, a clone (Bacteriophage Clone HUD-3) containing the 15 kb DNA fragment (Figure 1) isolated from the Maniatis's human DNA phage library in Example 1 hereinafter has been deposited at the National Collection of Industrial and Marine Bacteria (NCIMB: P.O. Box 31, 135 Abbey Road, Aberdeen AB9 8DG, Scotland, UK) under the Budapest Treaty, and the deposition date and number are as follows:

Date of deposition: February 27, 1989

Deposition number: NCIB 40122

Furthermore, clones in which individual $D_H$ genes in said 15kb fragment are integrated in appropriate vectors can be obtained also by cleaving and cloning using appropriate restriction enzyme cleavage sites located in the upstream and downstream of individual $D_H$ genes according to ordinary methods.

For the cloning, the above-mentioned E. coli can used as a host in the case where Charon 4A vector is used.

Furthermore, examples of the clones with the use of plasmid vectors include plasmid pPDXP (containing $D_{XP1}$ gene) obtained in Example 2 hereinafter, plasmid pDA (containing $D_{A4}$ gene), plasmid pDK (containing $D_{K1}$ gene), plasmid pDN (containing $D_{N4}$ gene), plasmid pDM (containing $D_{M2}$ gene) and plasmid pDLR (containing $D_{LR1}$ gene).

Further, E. coli MV1184 strains containing these plasmids pPDXP et al were deposited at the Fermentation Research Institute, Agency of Industrial Science and Technology (1-3 Higashi 1-chome, Tsukuba-shi, Ibaraki Prefecture, 305 Japan) under the Budapest Treaty on December 15, 1988 and the deposition numbers are as follows:

E. coli pPDXP (containing plasmid pPDXP)
FERM BP-2193

E. coli pDA (containing plasmid pDA)
FERM BP-2188

E. coli pDK (containing plasmid pDK)
FERM BP-2189

E. coli pDM (containing plasmid pDM)
FERM BP-2191

E. coli pDN (containing plasmid pDN)
FERM BP-2192

E. coli pDLR (containing plasmid pDLR)
FERM BP-2190

The nucleotide sequence of this 15kb DNA fragment is shown in Figure 2.

In the Figure 2, signal heptamer and signal nanomer portions are underlined and the open reading frame ($D_H$ coding regions) are boldly underlined.

Furthermore, 15 CAC(A/T)GTG region exist in regions other than adjacent regions of typical $D_H$ coding regions; they are underlined, too.

Among them, a heptamer located upstream of $D_M$ gene is placed between signal nonamers.

Six different $D_H$ genes were identified in the first 9kb region marked with circled a in Figure 1, i. e. 5'-$D_{XP4}$-(1061bp)-$D_{A4}$-(889bp)-$D_{K4}$-(1773bp)-$D_{N4}$-(430bp)-$D_{M1}$-(2610bp)-$D_{LR1}$-3'.

Characteristic 16bp repeating sequences exist between $D_{XP}$ and $D_{LR}$ and are repeated 21 times; the common repeating sequence is CCTGG(G/A)C(C/T)T(C/A)AC(C/T)TG(A/G) (a slash means "or"). Furthermore, the same 16bp sequences are repeated 17 times upstream of $D_{XP4}$.

In the first half portion of circled b retion of the 9 kb region following circled a region in Figure 1, a DNA fragment containing $D_{XP}$ gene is overlapped and $D_{XP1}$ and $D_{XP'1}$ exist.

The order of the sequence of $D_H$ genes in this circled b region is 5'-$D_{XP1}$-(97bp)-$D_{XP'1}$-(804bp)-$D_{A1}$-(884bp)-$D_{K1}$-(1426bp)-$D_{N1}$-(430bp)-Dx$M_2$-3'.

Furthermore, $D_{LR2}$ must exist as reported by Siebenlist et al. described above (Reference 3), although the present inventors have not yet confirmed its existence by sequence determination.

Furthermore, in comparison of repeating sequences of circled a and circled b regions in Figure 1, four large deleted and inserted portions exist; they are represented by circled I (256bp), circled II(859bp), circled III(499bp) and circled IV(16bp) and are boxed or marked with solid triangles (corresponding portions are also shown in Figure 1).

Furthermore, in portions with circled a and b, minute differences such as point mutations, deletion and insertion of 1-3 nucleotides are also observed (see Table 1 below).

$D_{XP4}$ gene upstream region of $D_{LR1}$ gene is locally overlapped in the region downstream of $D_{LR1}$ gene to form $D_{XP1}$ gene and $D_{XP'1}$ gene.

The hypothesis proposed in the above-mentioned report (Reference 4) that an unequal crossing (crossing-over) may occur in a homologous sequence CCACAG(C/')CCTCCC(C/T) has now been evidenced

by the fact described above.

As explained with Figures 1 and 2 above, since three $D_H$ gene families were newly found in the 15kb fragment cloned by the present inventors, the total number of $D_H$ genes present in the $D_H$ gene region can be estimated to be about 30.

Siebenlist et al. (Reference 3) reported that $D_H$ genes belonging to four $D_{LR}$ gene families are coded in series at 9kb intervals on a human genome. Recently, Matsuda et al. (Reference 7) identified additional $D_{LR}$ gene ($D_{LR5}$) in a $V_H$ gene region. Buluwel et al. (Reference 8) also described characteristics of constructions of large and small clusters of $D_H$ genes.

Further, the 15kb DNA fragment according to the present invention corresponds to the portion from the 3' end of $D_{LR4}$ gene to 5' end of $D_{LR2}$ gene described in these reports.

In order to estimate the total number of $D_H$ genes in Example 3, the present inventors prepared 6 probes each containing $D_H$ genes belonging to different families as shown in Figure 1)(clones each containing DNA fragments marked with <---> in Figure 1) and carried out Southern hybridization of human placenta DNA digested with BamHI, EcoRI and HindIII using these probes.

A $D_{LR}$ probe identified in the BamHI digest five bands located in positions corresponding to 18, 7.2, 6.5, 2.2 and 1.9 kb, which mostly agreed with the above-mentioned report in Reference 3. A $D_{XP}$ probe identified in the BamHI digest five bands located in positions corresponding to 20, 7.2, 6.5, 4.4 and 3.7 kb. Similarly, the $D_A$, $D_K$ and $D_M$ probes identified five bands in the digests with one of the three different restriction enzymes mentioned above.

The above-mentioned result demonstrates that these five $D_H$ gene families ($D_{LR}$, $D_{XP}$, $D_A$, $D_K$ and $D_M$) all comprise five members.

Further, with a $D_N$ probe, only three bands are identified in the digests with all three restriction enzymes mentioned above; this is probably because more than two $D_H$ genes are contained in one band since the 9kb repeating nucleotide sequences are closely resemble one another in the repeating regions. Furthermore, in addition, it is possible that large DNA fragments contain more than two $D_H$ genes.

Sizes of these individual probes are about 500 nucleotides long; one probe may possibly identify two bands responding to one $D_H$ gene since restriction enzyme cleavage sites are located in the regions covered with the probes.

Namely, it is possible that the above-mentioned factors interfere with estimation of numbers of $D_H$ genes by Southern hybridization.

However, it may safely be said that five repeating sequences located at 9kb intervals individually contain six different kinds of $D_H$ families and that the total number of the $D_H$ genes is about 30, since individual probes, except $D_N$ gene, identified 3 to 5 bands responding to three kinds of restriction enzymes.

On the other hand, as shown in Figure 4, individual $D_H$ genes in the above-mentioned 15 kb DNA fragment are classified into seven $D_H$ gene families.

As shown in the figure, each $D_H$ gene is flinked by a signal heptamer and nonamer (underlined) separated by 12 nucleotide spacer at both ends.

$D_{LR1}$ to $D_{LR4}$ are those disclosed by Siebenlist et al. in the above-mentioned report (Reference 3).

An analysis by the present inventors revealed that the nucleotides near the central region were not AA but GG. This is a new finding by the present inventors.

Furthermore, $D_{LR5}$ and $D_{Q52}$ are those reported in References 9 and 10, respectively.

As shown in the result of comparison demonstrated in Figure 4, signal heptamers are well conserved within the $D_H$ genes belonging to the same $D_H$ gene families. Yet, in place of CACAGTG at the 5' site of $D_{M2}$, CACAGCG was found, and TGCTGT was found at the 5' site of $D_{A1}$, respectively.

On the contrary, signal nonamers were relatively diverged from common nonamers (GGTTTTTGT or ACAAAAACC).

This phenomenon is observed generally in different signal regions of Ig and T-cell receptor (TRC) genes (Reference 11).

Furthermore, 12 mouse $D_H$ genes were classified into three families ($D_{SP2}$, $D_{FL16}$ and $D_{Q52}$) (Reference 2).

The largest family, $D_{SP2}$, contains the sequence TACTATGGT in the central region. Furthermore, the most frequently used $D_H$ gene, $D_{FL16.1}$, contains the sequence TACTACGGT. The $D_{SP2}$ family can code for Tyr-Tyr-Gly, Thr-Met and Leu-Trp and $D_{FL16.1}$ can code for Tyr-Tyr-Gly, Thr-Thr and Leu-Arg.

Further, most of mouse somatic cell $D_H$ sequences contain Tyr-Tyr-Gly (Reference 12).

This suggests that one among the three different open reading frames is mainly used in mice.

On the contrary, as shown in Figure 5, the three different reading frames are equally used in humans.

Further, sequences of 15 somatic-cell-type $D_H$ genes shown in Figure 5 are those published in Reference 4. Furthermore, besides the nucleotide sequences summarized in Figure 5, amino acid sequences of many human somatic-cell-type $D_H$ gene regions have been reported (Reference 12).

As shown in Figure 6, they are completely diverged except two combinations, namely POM and LAY consisting of the identical amino acids and MCE and NZU containing the identical amino acid sequence [RPPWRFT (Arg-Pro-Pro-Trp-Arg-Phe-Thr)].

None of other sequences has homology in sequences of more than three amino acids in length.

Subsequently, amino acid sequences of somatic-cell-type $D_H$ genes were compared as follows with those of embryonic-type $D_H$ genes determined from the nucleotide sequences shown in Figure 4.

On the assumption that all the three open reading frames can be read, cases where three out of four amino acids are identical or more than four amino acids are identical were searched.

Such attribution of somatic-cell-type $D_H$ to embryonic-type $D_H$ is adopted as the standard of judgment because it is very unlikely that three amino acids coincide casually. Their apparent amino acid sequences are extremely different one another; however, 19 somatic-cell-type $D_H$ sequences are all attributed to one of embryonic-type $D_H$ genes as shown in Figure 6.

Further, in Figure 6, the somatic cell type $D_H$ gene sequences are those reported in Reference 12 and the embryonic type $D_H$ gene sequences are those determined from the nucleotide sequences in Figure 2. Further, common amino acid portions are underlined. In Figure 6, individual alphabets represent amino acids as follows:

| | | | |
|---|---|---|---|
| A: Ala | C: Cys | D: Asp | E: Glu |
| F: Phe | G: Gly | H: His | I: Ile |
| K: Lys | L: Leu | M: Met | N: Asn |
| P: Pro | Q: Gln | R: Arg | S: Ser |
| T: Thr | V: Val | W: Trp | Y: Tyr |

The presence of identical sequences in different somatic-cell-type $D_H$ sequences found in MCE and NZU demonstrates that they are not produced by disordered insertion of nucleotides by TdT but encoded in embryonic-type sequences.

Nucleotide sequences expected to encode RPPWR are rich in GC. They seem to be encoded in DIR regions.

Since the 15 somatic-cell-type $D_H$ sequences disclosed are all attributed to any of embryonic-type $D_H$ genes or DIR genes at the DNA sequence level (Figure 5), it is less probable that many other $D_H$ genes, which are different from the seven $D_H$ gene families contained in the 15 kb DNA fragment according to the present invention, remain.

Somatic-cell-type $D_H$ genes which cannot be attributed to embryonic-type $D_H$ genes so far determined in Figure 6 may be derived from other $D_H$ genes belonging to the six $D_H$ genes, or divergence of the sequences may have been further extended by mutation of the somatic cells.

Consequently, it is convincing that the great diversity of human Ig H chains in the CDRIII regions is attributed to mutation of a limited number of embryonic-type $D_H$ genes and somatic cells.

These results demonstrate that somatic cell $D_H$ sequences of Ig H chain are made by the mechanism substantially the same in mouse and humans and that the central portion of somatic cell $D_H$ regions in $V_H$-$D_H$-$J_H$ structures are derived individually from unique $D_H$ genes.

On the other hand, in regard to revolution of $D_H$ genes, the following hypothesis has been suggested.

Sakano et al. made a deduction that DNA fragments coding for V and C domains were probably generated by multiplication of primary genes (Reference 14), and further Sakano et al. (Reference 13) suggested that ancestral V-encoding DNA fragments had been integrated but were devised into two portions, V and J genes, with insertion of a DNA element, i.e. an insertion fragment(IS)-like fragment, into these ancestral V genes.

Consequently, it is considered that 12-and 23-nucleotide spacer signals are present in both ends of the assumed IS-like DNA element and furthermore a similar IS-like fragment is once more inserted near the 5' end of the J gene so as to divide the ancestral gene into D and J genes.

Regularity in length of spacers in Ig or TCR gene portions can be explained by this hypothesis.

Furthermore, this IS-like DNA fragment may have been inserted in both directions.

In fact, $D_H$ genes found in Ig H chain gene portions in spiny dogfish contain the 12-nucleotide spacer

signal and 23-nucleotide spacer signal at the 5' end and 3' end, respectively (Reference 15).

If this IS hypothesis is correct, $D_{HQ52}$ located near the $J_H$ gene region should be the $D_H$ gene that evolved first.

Now, a question arises: whether other $D_H$ genes are derived from the $D_{HQ52}$ gene. However, in spite of the fact that frequency of mutation in signal regions is slightly low in intervening regions, it is unlikely that these six different $D_H$ genes are derived from the same primitive $D_H$ genes. Since substantial function of $D_H$ genes is to crease divergence, it is unreasonable that an evolutional selecting pressure which conserves nucleotide sequences, exists in the $D_H$ genes similarly in genes coding for ordinary proteins. Creation of $D_H$ genes without function may exert no influence on survivability.

Akira et al. (Reference 11) showed that heptamer and nonamer sequences having the same length as spacers were substantial for recombination, and nucleotide sequences in spacer regions were not important.

However, as Ichihara et al. (Reference 4) remarked, not only signal nonamer and heptamer sequences but also spacer regions are generally palindromic.

This characteristic can be considered as a reflection of the origin of $D_H$ genes but not as a result of mutation.

In regard to the above-mentioned speculations or hypothesis, new findings obtained in the present invention provide a key to the new evidence as follows:

Figure 7 is a summary of $D_H$ genes containing irregular spacer signals belonging to a new DIR gene family present in the 15 kb DNA fragment of the present invention.

As shown in Figure 7(a), CACAGTG sequence is located upstream of $D_M$ gene. This heptamer is flanked on both ends with signal nonamer-like sequences. T and A represent GGTTTTTGT- and ACAAAAACC-like sequences, respectively. The upper sequence ($DR_1$-$D_{M1}$) is present in the first 9kb region (a) in Figure 1 and the lower sequence ($DI_2$-$D_{M2}$) is present in the front half region (b) of the 9 kb region which follows (a) in Figure 1.

Nucleotides which are different within the DIR genes are marked with dots. Two somotic-cell-type $D_H$ sequences (HIG1 and 333) which are not attributable to any embryonic-type $D_H$ sequences have sequences homologous to these regions. The somotic-cell-type $D_H$ sequence of HIG1 has complementary sequences, and the somotic-cell-type $D_H$ sequence of 333 are homologous to the DIR gene in the same direction. Colons (:) demonstrate identical or complementary relations of nucleotides between the somotic-cell-type $D_H$ genes and the embryonic-type DIR genes.

The data of these somatic cell $D_H$ sequences are cited from References 16 for HIG1 and from Reference 17 for 333.

Furthermore, as shown in Figure 7(b), the $D_{IR}$ region is flanked on both ends each with a signal heptamer and a nonamer which are separated by 12 and 32 nucleotide spacers. Open squares represent GGTTTTTGT- and ACAAAAACC-like sequences. Closed squires represent signal heptamer sequences. The presumed $D_H$ genes are represented by open bars flanked at both ends each with a signal heptamer (closed triangles) and a nonamer (open triangles). Figures between the heptamer and nonamer show the length of spacers.

By the new identification of this DIR gene family, the followings can be suggested:

In Reference 4, the presence of another embryonic-type $D_H$ genes corresponding to somatic-cell $D_H$ sequences of HIG1 and 333 cells, which are rich in G and C residues, is predicted.

However, none of 17 embryonic-type $D_H$ genes which have been identified show homology to these somatic sequences and furthermore even in mice, none of several somatic-type $D_H$ sequences rich in G and C residues show homology to any of 12 embryonic-type $D_H$ fragments (Reference 2).

Alt and Baltimore (Reference 18) emphasized that sequences rich in GC appear in high frequency in N regions and that dG residues can be readily selected by exonucleotidyltransferase, when they proposed the involvement of the exonucleotidyltransferase in divergence of the N regions.

In the somotic-cell-type $D_H$ sequences rich in GC, which are not attributable to any one of such embryonic-type $D_H$ genes, regions encoded by embryonic-type $D_H$ genes are considered to be removed perhaps by exonuclease activity.

On the contrary, as shown in Figure 7(a), the present inventors found a DNA region which has a sequence complementary to the $HIGID_H$ sequence (18/21 nucleotides) and homologous to the 333 cell $D_H$ sequence (16/23 nucleotides) upstream of $D_M$ gene. As mentioned above, the regions which flank this DNA region contain several signal heptamers and nonamers. Their locations and the length of spacers are diagrammatically shown in Figure 7(b).

In spite of the fact that the distance from the heptamer at the 5' end in this DIR gene family to the heptamer in the $D_M$ gene is considerably long (127 or 151), this region is flanked on both ends with both 12-and 23-nucleotide spacer signals.

It is possible that this DIR region takes part in DIR-$D_H$ joining due to deletion or reversion.

Interestingly to say, polarity of homologous $D_H$ sequences in comparison with that of DIR is reverse for HIG1 but the same for 333. Since the supposed DIR-$D_H$ joining must have the 12- and 23- nucleotide spacer signal on the 5' side and the 12-nucleotide spacer signal on the 3' side, it may be a substrate to form any of the structures, $V_H$-DIR-$D_H$-$J_H$, $V_H$-$D_H$-DIR-$J_H$ and $V_H$-$D_H$-DIR-$D_H$-$J_H$.

Subsequently, in order to calculate frequency of mutation in $D_H$ genes and intervening regions based on the knowledge obtained by the present inventors, the portions marked with circled a and circled b in Figure 1 are compared and investigated.

To make $D_H$ genes functional fragment, the presence of 21-nucleotide spacer signals on both sides is considered to be substantial.

Frequency of mutations in the portions marked with circled a and circled b in Figure 1 is summarized in Table 1.

Table 1

| Intervening site[1)] | | Total | Identical | Not identical (1) | Not identical (2) | Deletion or insertion |
|---|---|---|---|---|---|---|
| I | 373- 775<br>10040-10446 | 409 | 358(87.5%) | 25 | 18 | 8 |
| II | 863- 1573<br>10718-11429 | 720 | 626(86.9%) | 54 | 23 | 17 |
| II' | 1830 -1923<br>11430-11521 | 94 | 85(90.4%) | 6 | 1 | 2 |
| III | 1996- 2884<br>11594-12477 | 895 | 802(89.6%) | 53 | 23 | 17 |
| IV | 2961- 3091<br>12557-12685 | 133 | 124(93.2%) | 1 | 2 | 6 |
| IV' | 3092- 3138<br>13185-13231 | 48 | 40(83.3%) | 4 | 2 | 2 |
| IV'' | 3139- 3535<br>13248-13642 | 400 | 327(81.8%) | 26 | 39 | 8 |
| IV''' | 4395- 4733<br>13643-13982 | 341 | 295(86.5%) | 26 | 17 | 3 |
| V | 4808- 5237<br>14060-14489 | 432 | 388(89.8%) | 23 | 15 | 4 |
| VI | 5311- 5667<br>14563-14918 | 355 | 321(90.4%) | 16 | 15 | 3 |
| | | | | 233 | 152 | 70 |

1) Intervening sites, I-VI, correspond to I-VI in Figure 1.

Further, four long inserted or deleted regions (portions marked with circled I and IV in Figures 1 and 2) are not included in this comparison.

In Table 1, group I corresponds to a region between the 16-bp repeating region and $D_{XP}$ gene and group II corresponds to a region between the $D_{XP}$ gene, and the $D_A$ gene.

Furthermore, inserted regions from 1574 to 1829, from 12686 to 13184, from 13232 to 13247 and from 3536 to 43 94 are not included in comparison shown in Table 1.

Furthermore, group III corresponds to a region between $D_A$ gene and $D_K$ gene, and group IV corresponds to a region between $D_K$ gene and $D_N$ gene.

On the other hand, group V corresponds to a region between $D_N$ gene and $D_M$ gene and group VI corresponds to downstream of $D_M$ gene.

(1) and (2) in the column marked "Not identical" represent individual base transformations (G ←→ A and T ←→ C) and base group transformations within base groups (purine base ←→ pyrimidine base), respectively. Further, the figures are log numbers of bases.

As shown in Table 1, a ratio of base transformation (1) : (2) was about 3:2.

Furthermore, the deletion (or insertion) of short nucleotides totaled 45, 8 and 3 times for 1bp, 2bp and 3bp nucleotides. respectively.

Percentage of difference in sequences of two overlapping units in intervening regions totals about 12 %.

In signal regions, differences in nucleotides in two identical genes were 7bp (8%) for nonamers, 4bp (6%) for heptamers and 10bp (8%) for spacer regions.

Most of the mutations in signal regions and the intervening regions are considered to be attributed to errors in DNA replication.

In summary, inconsistency were observed 236 times in a single nucleotide, 52 times in consecutive two nucleotides, 7 times in three consecutive nucleotides, once in four consecutive nucleotides and once in eight consecutive nucleotides.

Most of the deletions or insertions of 1 to 3 nucleotides occur due to slippage reactions (Reference 21) by DNA polymerase, such as from GCCC to GCC or GCCCC.

Some of the mutations observed in $D_H$ coding regions are the same as described above.

There is no difference in nucleotides between $D_{A4}$ coding region and $D_{A1}$ coding region whereas inconsistency in a single nucleotide between $D_{M1}$ coding region and $D_{M2}$ coding region was observed in two sites.

The deletions (or insertions) of three successive nucleotides between the $D_{N1}$ coding region and the $D_{N4}$ coding region may be attributable to the slippage reaction by DNA polymerase. For one AGC sequence among three repeating AGC sequences was deleted.

For example, differences observed in other $D_H$ coding regions such as between $D_{XP1}$, and $D_{XP,1}$, between $D_{K4}$ and $D_{R1}$, and within $D_R$ family genes, have different characteristics.

Mutated nucleotides are crowded in limited areas; diverged nucleotides show homology to other $D_H$ coding regions.

In reference 4, it was remarked that coding sequences of $D_H$ genes seemed to be constructed with short oligonucleotides and that diverse sequences such as GATATT in $D_{XP1}$ and GGGGA in $D_{XP,1}$, were observed also in $D_{LR1}$ and $D_{HQ52}$, respectively.

Consequently, gene transformation (Reference 19) may be one of major routes that increase divergence in the $D_H$ coding regions.

Furthermore, $D_H$ sequences in mouse and humans were compared using the new knowledge in the present invention described above.

As a result, as shown in Figure 8(a), it was revealed that $D_H$ coding and signal sequences in one human $D_H$ genes showed 81% (59/73) homology to those of mouse $D_{SP2}$, and that additionally in the case of $D_{K4}$, sequences not only in $D_H$ gene region but also in the adjacent regions at the 5' site and the 3' site have 57% (135/236) homology to those of $D_{SP2}$ (Figure 8(b)).

This suggests that no extensive mutations occurred in any $D_H$ gene portions once the $D_H$ had evolved.

These adjacent sequences seemed to be greatly diverged within the six adjacent $D_H$ genes; however, in some combinations, 56 to 61 % homology was found in the extent of 100 nucleotides of adjacent sequences on the 5' sites.

The same extent (52 to 61%) of complementary relations was found between the 5' and 3' adjacent sequences (Figure 9(a)).

Interesting to say, sequences of complementary adjacent regions belong to different gene families each other as shown in Figure 9(b).

The above-mentioned observations are summarized as follows:

a. difference in the intervening sequences between the two units generated by overlapping is about 12 % but difference in signal regions is 6 to 8 %;

b. about 60 % of homology is observed between the sequences at the 5' adjacent regions in a certain

combination;

c. also, about 60 % of complementary relation is observed between sequences in the 5'- and 3-adjacent regions;

d. furthermore, CAC(A/T)GTG-like sequences were found in many of $D_H$ coding regions (Figure 4).

On the basis of these observations, the present inventors can propose the following hypothesis regarding the origin of $D_H$ genes.

A cluster of the primordial half D gene (prih-D) having a structure shown in Figure 10 was present.

Nucleotides sequences of the prih-D gene and those in the regions surrounding this gene had been diverged from the beginning.

Ricombinase of the V-(D)-J joint recognized two prih-D genes.

Polarity of these DNA fragments were the same so that reversion occurred in place of deletion.

N fragment may have been inserted between two signal heptamers.

The resulting fragment is flanked on both ends with two pairs of 12-nucleotide spacer signals so as to function as $D_H$ genes.

This hypothesis was evidenced several times in this limited region.

A gene which is assumed to be the origin of this prih-D gene should contain signals, consist of multiple gene families, have been diverged and form a cluster.

This prih-D gene may be the 3' end portion of V gene, for homology has been found between the sequences in the adjacent regions of $D_H$ gene and that in the 3' adjacent regions of $V_H$ gene by the present inventors (Figure 11).

The extent of the homology or complimentarity (57 to 63 %) was very similar to that observed between adjacent regions of the $D_H$ gene.

To transform the $V_H$ gene to the prih-D gene, the sequence CAC(A/T)GTG must have been created in the 23-nucleotide spacer region.

It is considered that this could have happened. Since the CAC(A/T)GTG-like sequences are found in the spacers at the 3' side of the $V_H$ gene even at present (Reference 20).

This model is advantageous because genes obtained by transversion are diverged $D_H$ genes having functions and because it is not necessarily assumed that furthermore mutations had been occurred in order to obtain various kinds of $D_H$ gene segments.

From the above-mentioned various knowledges, it is revealed that nucleotide sequences of adjacent regions of V-D-J junctions in the heavy chain of human immunoglobulin and TCR beta-chain and delta-chain genes, namely V(-N-C-N-)J, have substantially unlimited divergence.

Further, N denotes a nucleotide fragment which is not encoded in embryonic-type V, D or J genes and is inserted into a DNA sequence by terminal transnsferase during the process of V-D-J rearrangement.

For example, the $V_H$-$D_H$-$J_H$ rearrangement junction site of the DNA clone lambda IGD-1 is shown in Figure 12, which is obtained by hydrolizing DNAs of IgD-secreting myeloma cells derived from the bone marrow of a patient described later, detecting 20kb DNA with a $J_H$ probe, isolating and packaging the DNA into a phage so as to make DNA clone lambda IGD-1 (Reference 26).

It is revealed that only $D_{K1}$ and $D_{XP,1}$ are contained in the $D_H$ portion (Figure 13).

Namely, in each somatic-type $D_H$ gene as in all other cases, just a portion of genes among a large number of embryonic-type $D_H$ genes contained in DNA fragments such as the 15kb DNA which is cloned in the present invention is used at random for $V_H$-$D_H$-$J_H$ rearrangement, which can demonstrate unlimited divergence in nucleotide sequences of somatic-type cell $D_H$ genes.

A peptide portion of antibodies corresponding to the DNA fragment with this unlimited divergence is the CDRIII region.

On the other hand, lymphocyte-related tumor cells of individuals are known to be monoclonal and considered to have DNA fragments, characteristic to individual tumors, in $D_H$ region and $D_H$-adjacent regions ($N_H$).

Accordingly, if nucleotide sequences of DNA fragments in $D_H$ regions and $D_H$-adjacent regions ($N_H$), characteristic to a patient with lymphocyte-related tumor, are determined as tumor markers, ratios of the tumor cells (amount of DNA) to whole lymphocytes (amount of DNA) of the patient can be calculated using DNA probes or the like carrying these sequences.

Currently, in B-lymphocyte-related tumors T-lymphocyte-related tumor, leukemia, myeloma or the like, chemotherapy using various anticancer agents and steroid agents is carried out to suppress bonemarrow formation. However, it is almost impossible to completely destroy tumor cells using such agents for chemotherapy because sufficient doses cannot thoroughly be administered owing to strict precautions against possible side effects.

Consequently, it is desirable and in fact practiced to regularly test and diagnose changes of the state of

tumor cells included in regenerating normal bornmarrow cells even in a so-called remission period of lymphoma or leukemia, in which leukemic activity can be temporarily suppressed, so as to diagnose, prevent and treat recurrence of leukemia or the like in an early stage; however, diagnostic methods available at present are fully depend on morphological analyses such as cytodiagnosis and bonemarrow examination by bonemarrow stabbing.

In the bonemarrow examinations, diagnosis of recurrence is possible only when the number of leukemic cells exceeds 5 to 15 % and thus precise diagnosis in a early stage is difficult; further, diagnosis by cell fluorescence analysis using monoclonal antibodies specific to lymphocytes is recently carried out, but the detectability is not much improved.

Accordingly, based on the above-mentioned knowledge, the present inventors have established a DNA diagnostic method in which a ratio of the number of DNA of tumor cells to that of normal lymphoid cells can be calculated by identifying sequences of DNA fragments characteristic to tumor cells, using unlimited divergence of immunoglobulins produced by lymphoid cells, DNA fragments of $V_H$ ($N_H1$-$D_H$-$N_H2$) $J_H$ in rearranged genes corresponding to TCR, particularly nucleotide sequences corresponding to CDRIII of $D_H$ region and regions adjacent to the $D_H$ region ($N_H1$ and $N_H2$).

This method of DNA diagnosis includes the following steps:

First, DNA sequences characteristic to lymphoid cells of individual patients are determined; DNA fragments which are conserved and found in most of all cells of $V_H$ and $J_H$ genes, namely an oligonucleotide in $V_H$, is selected as the first primer and an oligonucleotide complementary to an oligonucleotide in $J_H$ is synthesized as the second primer, in determining the amount of lymphoid neoplasm cells; these primers are mixed with DNA fragments fractionated from lymphoid neoplasm cells of the patients; a large amount of DNA are proliferated in the presence of DNA polymerase by the PCR method (the polymerase chain reaction method by Saiki et al.: Reference 24) or the like; nucleotide sequences of genes ($N_H1$ and $N_H2$) characteristic to the lymphoid cells of the individual patients are determined; and $V_H$-(a)-$N_H1$(a) DNA fragment comprising DNA fragment $V_H$(a) selected from the above-mentioned $V_H$ and a DNA fragment $N_H1$(a) selected from the determined $N_H1$, or, in case of need, a DNA fragment extended to $D_H$ region, are synthesized as the third primer characteristic to the patients. Furthermore, a $V_H2$(a)-$J_H$ (a) DNA fragment comprising a DNA fragment $J_H$(a) which is complementary to a DNA fragment selected from the above-mentioned $J_H$ and a DNA fragment $N_H2$(a) which is complementary to a DNA fragment selected from determined $N_H2$, or, in case of need, a DNA fragment complementary to a DNA fragment in other portions of $J_H$ region, is synthesized as the fourth primer characteristic to the patients.

Subsequently, at intervals in the cource of the therapy, patient's lymphoid DNAs are mixed with a pair of the third primer and the second primer (or the fourth primer) or a pair of the first and the fourth primer, and then a large number of DNA are proliferated in the presence of DNA polymerase by the PCR method; thus, the ratio of lymphoid neoplasm cells is calculated from the amount of DNA thus produced.

Using this method, evaluation of therapeutic effect of anticancer agents applied to patients with leukemia or the like, diagnosis of efficacy and diagnosis of recurrence can be readily carried out in an early stage.

This diagnostic method can be preferably used particularly in diagnosis of B-lymphoid neoplasm, T-lymphoid neoplasm, acute leukemia, myeloma and the like.

An example of the diagnostic method of the present invention in a patient with IgD-secreting myeloma will be demonstrated as follow:

First, a nucleotide sequence of a DNA fragment of rearrangement junction region of $V(N^1\text{-}D\text{-}N^2)J$ characteristic to the patient is determined.

DNAs are prepared from bonemarrow cells or peripheral blood lymphocytes of the patient with IgD-secreting myeloma according to the conventional method (Reference 22); DNA fragment oligonucleotides which are highly conserved in $V_H$ are selected as the first primers; further, DNA fragment oligonucleotides carrying sequences complementary DNA fragment oligonucleotides which are highly conserved in $J_H$ are selected as the second primers; and the selected sequences are synthesized using Applied Biosystems Model 380A DNA synthesizer, for example, according to the solid phase triester method (Reference 23).

Subsequently, the synthesized oligonucleotides are purified by HPLC.

The followings can be used as the first and second primers:

The first primers:

5'-CACGGCCGTGTATTACTGTG-3'

5'-CACGGCCATCTATTTTTGTG-3'

$$5'\text{-CC}\begin{pmatrix}A\\T\\C\end{pmatrix}\text{GG}\begin{pmatrix}A\\G\end{pmatrix}\begin{pmatrix}C\\A\end{pmatrix}\text{G}\begin{pmatrix}A\\G\end{pmatrix}\text{GG}\begin{pmatrix}A\\G\\C\\T\end{pmatrix}\text{CT}\begin{pmatrix}A\\G\end{pmatrix}\text{GA}\begin{pmatrix}G\\A\end{pmatrix}\text{TGG-3}'$$

$$5'\text{-CC}\begin{pmatrix}A\\T\\C\end{pmatrix}\text{GG}\begin{pmatrix}A\\G\end{pmatrix}\begin{pmatrix}C\\A\end{pmatrix}\text{A}\begin{pmatrix}A\\G\end{pmatrix}\text{GG}\begin{pmatrix}A\\G\\C\\T\end{pmatrix}\text{C T}\begin{pmatrix}T\\G\end{pmatrix}\text{GA}\begin{pmatrix}G\\A\end{pmatrix}\text{TGG-3}'$$

The second primer:
5'-ACCTGAGGAGACGCTGAC-3'

Subsequently, using DNAs prepared according to the conventional method from lymphoid cells of an IgD-secreting myeloma patient to be diagnosed and the first and second primers, a large numbers of a DNA sequence [$V_H$-($N_H$1-$D_H$-$N_H$2)-$J_H$ which codes for[V-($N^1$-D-$N^2$)-J] are multiplied using the PCR method.

A nucleotide sequence of the multiplied DNA sequences is determined by the dideoxy method by Sanger et al. (Reference 25) and then the DNA sequence is registered as a tumor marker characteristic to the patient.

Subsequently, nucleotide sequences of genes ($N^1_H$, $N^2_H$) adjacent to the $D_H$ region characteristic to the lymphoid cells of this patient is selected, and a DNA fragment $V_H^{(a)}$-$N_H^{1(a)}$ comprising an arbitrary DNA fragment $V_H^{(a)}$ in the above-mentioned $V_H$ and an arbitrary DNA fragment $N_H^{1(a)}$ in the determined $N_H$1, or in case of need, a DNA fragment extended to $D_H$, is synthesized as the third primer characteristic to the patient.

Furthermore, a $N_H$2(a)-J(a) DNA fragment consisting of a DNA fragment $J_H$(a) which is complimentary to the arbitrary DNA fragment in the above-mentioned $J_H$ and a DNA fragment $N_H$2(a) which is complementary to an arbitrary DNA fragment in a determined $N_H$2, or in case of need, a DNA fragment complementary to a DNA fragment in other portions of $J_H$ region are synthesized as the fourth primer characteristic to the patient.

For example, the followings can be used as the third and fourth primers:
The third primer

5'-TGTGCAAGAGGGCCTTTGAA-3'
The fourth primer

5'-CAGGCCCAAGAGTGGGCATT-3'

Subsequently, the third and fourth primer and DNAs prepared according to the conventional method (Reference 22) from lymphoid cells of the IgD-secreting myeloma patient to be diagnosed, in course of time during treatment, are treated by the PCR method, and then the presence of DNA sequences amplified by the action of these primers is examined or a percentage of tumor cells is determined from the amount of replicated DNA.

Further, in this replication process, a combination of the first primer and the four primer or that of the third primer and the fourth primer can also be used.

Detection of replicated DNA sequences can be carried out by developing a reaction mixture by electrophoresis and comparing bands, visualized by color reaction or the like, with those of DNA sequences, similarly treated, which had been registered as a tumor marker characteristic to the patient so as to examine whether the bands appear in the same location.

Furthermore, the bands can be analyzed by Southern hybridization using DNA fragments used as primers as probes.

Further, the third and fourth primers are arbitrarily selected from the DNA sequences registered as tumor markers characteristic to individual patients; the primers are repeatedly treated in the same manner as described above with every selected sequence and determined by confirming their efficacy. The primers are not limited to the above-mentioned sequences.
[Examples]

Example 1

Clone HUD-3 of human embryonic-type DNA was isolated from a human phage library (Reference 5) provided by Maniatis (Harvard University) according to the method described by Ichihara et al. (Ichihara. Y., Kurosawa, Y. et al.: Eur. J. Immunol., 18 , 649-652, 1988).

Namely, clone HUD-3 containing a 15kb DNA fragment which hybridizes with a $D_H$-$J_H$ probe prepared by Ravetch et al. (Reference 10) was isolated from the above-mentioned Maniatis's human DNA phage library using the $D_H$-$J_H$ probe by the Benton-Davis method (Reference 6).

Then, the restriction endonuclease cleavage map for the 15kb fragment in the resultant clone HUD-3 was made according to the conventional method. The nucleotide sequence of this fragment was determined by the dideoxy method (Sanger, F. et al.: Proc. Natl. Acad. Sci., U.S.A., 74 , 5463-5467, 1977) using M13mp10 or M13mp11 as a cloning vector according to the method of Messing et al. (Messing, J. et al.: Nucleic Acids Res., 9 , 309-321, 1981). The restriction endonuclease cleavage map and the nucleotide sequence thus obtained are shown in Figures 1 and 2, respectively.

From the results shown in Figures 1 and 2, it was confirmed that the 15 kb fragment of clone HUD-3 contains $D_{LR1}$ corresponding to $D_1$ in Reference 3 and that the 15 kb fragment contains a embryonic-type $D_H$ gene region.

Example 2

DNA fragments were prepared from the 15 kb fragment in clone HUD-3 using the various restriction endonuclease cleavage sites shown below. Each of the resultant fragments was independently cloned according to the conventional method in a BLUESCRIPT KS plus vector (Stratagene, San Diego, CA, U.S.A.). Recombinant DNAs carrying sites marked with ←→ in Figure 1, i.e. plasmid pPDXP (containing $Dxp_1$ gene), plasmid pDA (containing $D_{A4}$ gene), plasmid pDK (containing $D_{K1}$ gene), plasmid pDN (containing $D_{N4}$ gene), plasmid pDM (containing $D_{M2}$ gene) and plasmid pDLR (containing $D_{LR1}$ gene), were obtained.

A fragment containing $D_{xp1}$ gene (containing $D_{xp.1}$ gene):

BamHI-BalI*(10232)-PstI(10749)

A fragment containing $D_{A4}$:

BamHI-EcoRV*(1561)-AccI(2287)-XhoI

A fragment containing $D_{K1}$ gene:

SacI(12241)-SmaI(12875)

A fragment containing $DN_4$ gene:

BamHI(4260)-NcoI* (4901)-SalI

A fragment containing $D_2$ gene (including $DIR_2$:

XbaI-StuI* (14339)-EcoI(14923)

A fragment containing $D_{LR1}$ gene:

XbaI-NcoI(7577)-HindIII(8174)

Further, "*" above represents a site which was destroyed during constructing processes, and the figures in parentheses correspond to the nucleotide numbers shown in Figure 2.

Further, in isolating the plasmids containing individual $D_H$ genes, the polylinker sites in the vectors were used.

Each of the recombinant DNAs thus obtained was independently introduced into E. coli MV1184 by the conventional method.

Example 3

Using each of the fragments obtained from the 15 kb fragment in Example 2 as a probe, Southern hybridization of human placenta DNA was carried out.

Human placenta DNA was extracted according to the method of Cross-Bellard et al. (Gross-Bellard, M. et al.: Eur. J. Biochem., 36 , 32-38, 1973).

The extracted human placenta DNA was digested with restriction endonucleases, BamHI, EcoRI and HindIII.

Three kinds of DNA mixtures thus obtained (5 microns each) were independently subjected to electrophoresis using 0.8 % agarose gel.

Each gel was treated first in 0.25M HCl for 15 minutes, then with 0.5M NaOH and 1.5M NaCl for 30 minutes, and further, in a 1.0 M Tris-HCl buffer solution (pH 7.5; containing 1.5 M NaCl) for 30 minutes.

Each of the resulting DNAs obtained by this treatment was transferred onto a nylon membrane (Hybond N, a product of Amersham) with 20 x SSC using LKB 2016 VacuGene vacuum blotting system (a product of Pharmacia) according to the modified method of Oliszewska et al. (Olszewska, S. et al.: Trends in Genetics, 4 , 92-94, 1988) of Southern's method (Southern, E., M.: J. Mol. Biol., 98 , 503-517,1975).

The DNA thus transferred onto the membrane was fixed by radiation of ultraviolet rays for 5 minutes and heat treatment at 80° C for 2 hours.

Each membrane prepared by the above-mentioned procedure was immersed in a boiling washing solution (0.1 x SSC, 0.1% SDS) for 15 minutes and then subjected to prehybridization [in which the membrane was treated overnight at 42° C in 5 x SSPE (20 x SSPE: 0.2M $NaH_2PO_4$ buffer solution (pH 7.4, containing 3.6M NaCl and 20 mM EDTA), 0.1 mg/ml heat-denatured sermon sperm DNA, 50% formamide (a product of Merck (Darmstat, West Germany), 5% Irish cream liqueur (a product of Baileys, Dablin, Ireland) and 0.1% SDS).

After completion of this prehybridization procedure, hybridization was carried out in the same manner as described above, except that heat-denatured $^{32}P$-labeled DNAs, prepared by a method including a step for labeling the DNA fragments, obtained in Example 2, with $^{32}P$ according to the random oligonucleotide labeling method (Feinberg, A. P. et al.: Anal. Biochem., 132, 6-13, 1983), were added to the individual probes.

Washing at 42° C with 4 x SSC for 30 minutes and fuaarther washing twice with 0.5 x SSC were carried out; then autoradiography was carried out at -80° C.

The result is shown in Figure 3.

Example 4

[selection of sequences to be used for the first and second primers]

Using the DNA fragments obtained in Examples 1 and 2 and the various probes used in cloning of these DNA fragments, DNAs prepared from bonemarrow cells or peripheral blood lymphatocyte cells of an IgD-secreting myeloma patient according to the ordinary method (Reference 22) were subjected to Southern hybridization in the same manner as described in Example 3, DNA fragments having rearrangement junction of $V_H(N_H1\text{-}D_H\text{-}N_H2)J_H$ were specified and then cloned by the method of Benton-Davis used in Example 1; the nucleotide sequences of these fragments were analyzed by the dideoxy method of Sanger et al. (Reference 25).

Subsequently, DNA sequences, which are conserved in high frequency respectively in $V_H$ gene region and $J_H$ gene region in the region coding for the rearrangement junction of $Y(N^1\text{-}D\text{-}N^2)J$, $V_H(N_H1\text{-}D_H\text{-}N_H2)J_H$, thus obtained, were specified.

As a result, as DNA sequences which are conserved in high frequency in $V_H$ gene region, the following sequences (any one in parentheses may be selected and different ones within the parentheses may be used in a mixture) were found and used for sequences of the first primers:

(a) 5'-CACGGcCGTGTATTACTGTG-3'

(b) 5'-CACGGCCATCTATTTTTGTG-3'

$$\text{(c)}\ 5'\text{-CC}\begin{pmatrix}A\\T\\C\end{pmatrix}\text{GG}\begin{pmatrix}A\\G\end{pmatrix}\begin{pmatrix}C\\A\end{pmatrix}\text{G}\begin{pmatrix}A\\G\end{pmatrix}\text{GG}\begin{pmatrix}A\\G\\C\\T\end{pmatrix}\text{CT}\begin{pmatrix}T\\G\end{pmatrix}\text{GA}\begin{pmatrix}G\\A\end{pmatrix}\text{TGG-3}'$$

$$\text{(d)}\ 5'\text{-CC}\begin{pmatrix}A\\T\\C\end{pmatrix}\text{GG}\begin{pmatrix}A\\G\end{pmatrix}\begin{pmatrix}C\\A\end{pmatrix}\text{A}\begin{pmatrix}A\\G\end{pmatrix}\text{GG}\begin{pmatrix}A\\G\\C\\T\end{pmatrix}\text{CT}\begin{pmatrix}T\\G\end{pmatrix}\text{GA}\begin{pmatrix}G\\A\end{pmatrix}\text{TGG-3}'$$

Furthermore, as sequences complementary to the DNA sequences conserved in high frequency in $J_H$ gene region, the following sequences were found and used for sequences of the second primers:

5,-ACCTGAGGAGACGGTGAC-3'

5,-ACCTGAGGAGACGCTGAC-3'

Further, for reference, Figure 14 shows a part of the sequence in $J_H$ gene region which was obtained as a result of the above-mentioned analysis used for selection of the sequences of the second primers.

Example 5

One of the sequences for the first primers selected in Example 4 was selected, and oligonucleotides carrying this sequence was synthesized by the solid-phase triester method (Reference 23) using Applied Biosystems Model 380 DNA synthesizer and then purified using HPLC (high performance liquid chromatography).

Separately, the oligonucleotides carrying the sequences for the second primers selected in Example 4 were synthesized by the solid-phase triester method (Reference 23) using Applied Biosystems Model 380 DNA synthesizer and then purified using HPLC (high performance liquid chromatography).

Subsequently, in the same manner as described in Example 4, DNA of bonemarrow cells or tumor cells fractionated from periphery blood lymphatic cells of IgD-secreting myeloma patient (45-year-old female) to be diagnosed and the above-mentioned two kinds of purified oligonucleotides were reacted by the PCR method of Saiki et al. (Reference 24).

The composition of a reaction solution is given below. The reaction conditions were those by Saiki et al., and the annealing temperature was 55 °C. Further, the reaction for DNA replication was repeated 25 times. Composition of reaction solution:

| | |
|---|---|
| Cellular DNA (100 micrograms/ml) | 10 microliters |
| First primer (20 micrograms/ml) | 3 microliters |
| Second primer (20 micrograms/ml) | 1 microliter |
| dNTP (150 micromols) | 16 microliters |
| Taq polymerase (10 u/microliter) | 1 microliter |
| 10 x buffer solution for PCR | 10 microliters |
| Distilled water | 59 microliters |
| Total | 100 microliters |

(Buffer solution for PCR: Tris-HCl (pH 8.0), 50 mM NaCl, 10 mM $MgCl_2$, 10 mM mercaptoethanol)

After the completion of the replication reaction, the reaction mixture was developed by electrophoresis using agarose gel and then DNA fragments were stained with ethidium bromide.

As a result, a band located at position 282bp was detected.

Further, when, the sequence (c) or (d) of the first primers selected in the Example 4 was used, a single band at position 282bp was detected; in the case of this IgD-secreting myeloma patient, the sequences (c) and (d) were better reacted than the sequences (a) and (b).

Subsequently, this DNA fragment of 282 bp was treated at needs with appropriate restriction endonucleases, and its nucleotide sequence was determined by the dideoxy method used in the Example 1.

By analyzing the resultant nucleotide sequence, it was confirmed that a sequence similar to those shown in Figure 12 was contained.

This nucleotide sequence was registered as a DNA fragment marker characteristic to the above-mentioned patient to be diagnosed.

Example 6

[Preparation of the third and fourth primers]

From $V_H$ ($N_H1$-$D_H$ region of the sequence shown in Figure 12, the following sequence was selected, and an oligonucleotide having this sequence was synthesized and purified as a sequence for the third primer in the same manner as used in Example 4:

5'-TGTGCAAGAGGGCCTTTGAA-3'

Furthermore, a sequence, which was located further downstream of the sequence complimentary to the sequence for the second primer downstream of the sequence of $D_H$-$N_H2)J_H$ region shown in Figure 12, was selected, and an oligonucleotide having the following sequence complementary to this selected sequence was synthesized and purified as a sequence for the fourth primer in the same manner as used in

Example 4:

5'-CAGGCCCAAGAGTGGGCATT-3'

Example 7

[Sensitivity test of the diagnostic method of the present invention]

An amount of DNA per cell in the tumor cells fractionated from the patient to be diagnosed in Example 5 was determined.

Subsequently, from DNAs fractionated from normal placenta cells and normal lymphoid cells by the method used in Example 4, DNAs corresponding to cell numbers of $10^2$,$10^4$ and $10^6$ were prepared.

Subsequently, the tumor cell DNA and the normal placenta cell DNA or normal lymphoid cell DNA were mixed, and then samples A to I having the following ratios of cell numbers (x = number of tumor cell DNA, Y = number of normal placenta cell DNA or normal lymphoid cell DNA) were prepared. The amount of total DNA in the individual sampels (total) were adjusted to be constant before carrying out PCR.

Sample A: Tumor cell DNA only;
Sample B Normal placenta cell DNA only;
Sample C Normal placenta cell DNA was used; X : Y = $10^{-2}$ : 1 (X = 2ng)
Sample D Normal placenta cell DNA was used; X : Y = $10^{-4}$ : 1 (X = 0.2pg)
Sample E Normal placenta cell DNA was used; X : Y = $10^{-6}$ : 1 (X = 0:2pg)
Sample F Normal lymphoid cell DNA only;
Sample G Normal lymphoid cell DNA was used; X : Y = $10^{-2}$ : 1 (x = 2ng)
Sample H Normal placenta cell DNA was used; X : Y = $10^{-4}$ : 1 (X = 20pg)
Sample I Normal placenta cell DNA was used; X : Y = $10^{-6}$ : 1 (X = 0.2pg)

Subsequently, using the individual samples, DNA replication with the third and the fourth primers prepared in Example 5 was carried out by the PCR method

Composition of the reaction solution for PCR was as follows: Sample volumes are 11.5 microliters for samples A to E and 16.2 microliters for samples F to I. The concentration of each constituent was the same as used in

Example 5.

Composition of reaction solution for PCR

| | |
|---|---|
| Sample | 11.5 or 16.2 microliters |
| First primer | 3 microliters |
| Second primer | 3.5 microliters |
| dNTP | 16 microliters |
| Taq polymerase | 0.5 microliter |
| 10 x buffer solution for PCR | 10 microliters |
| Distilled water | 55.5 or 50.8 microliters |
| Total | 100 microliters |

Conditions for the reaction was by Saiki et al. and an annealing temperature was 65° C. Reaction for DNA replication was repeated 50 times.

After completion of the replication reaction, the reaction mixture was developed by electrophoresis using an agarose gel, and then the resultant DNA fragments were stained with ethidium bromide.

A single band at position 131bp was each detected in samples A, C to E and G to H.

No band was detected either in sample B or F.

Subsequently, a DNA fragment of 131 bp was isolated by the conventional method, and treated at needs with restriction endonucleases, and then its nucleotide sequence was determined by the dideoxy method used in

Example 1.

By analyzing the obtained nucleotide sequence, it is confirmed that the 131bp DNA fragment was contained in the sequence shown in Figure 12.

From these results, it was confirmed that detection was possible up to a tumor cell concentration of $1/10^6$.

Example 8

In the course of treatment of the patient to be diagnosed in Example 5, lymphatic cells were fractionated at intervals, DNAs were isolated from the obtained cells, and a percentage of tumor cells contained in lymphatic cells were determined by the method used in Example 7.

As a result, it was confirmed that, after the treatment, increasing tumor cell numbers were again decreased with time by administration of an anticancer agent.

References

1. Kurosawa, Y.: Protein, Nucleic Acid and Enzyme, 31 , 756-768, 1986.
2. Kurosawa, Y. and Tonegawa, S.: J. Exp. Med., 155 , 201-218, 1982.
3. Siebenlist, U. et al.: Nature, 294, 631-635, 1981.
4. Ichihara, Y., Kurosawa, Y. et al.: Eur. J. Immunol., 18, 649-652, 1988.
5. Lawn, R. M. et al.: Cell, 15 , 1157-1174, 1978.
6. Benton, W. D. and Davis, R. W.: Science, 196, 180-182, 1977.
7. Matsuda, F. et al.: EMBO J., 7 , 1047-1051, 1988.
8. Buluwela, L. et al.: EMBO J., 7 , 2003-2010, 1988.
9. Zang, S. Q. et al: Gene, 33 , 103-119, 1985.

10. Ravetch, J. v. et al.: Cell, 27 , 583-591, 1981.
11. Akira, S. et al.: Science, 238, 1134-1138, 1987.
12. Kabat, e. A. et al.: Sequences of Proteins of Immunological Interest, NIH, 1987.
13. Sakano, H. et al.: Nature, 280, 288-294, 1979a.
14. Sakano, H. et al.: Nature, 277, 627-633, 1979b.
15. Hinds, K. R. et al.: Nature, 320, 546-549, 1986.
16. Kudo, A. et al.: Gene, 33, 181-189, 1985.
17. Cleary, M. L. et al.: Cell, 47 , 19-28, 1986.
18. Alt, F. is. et al.: Proc. Natl. Acid. Sci. USA, 79 , 4118-4122, 1982.
19. Baltimore, D.: Cell, 33 , 592-593, 1981.
20. Kodaira, M. et al.: J. Mol. Biol., 190, 529-541, 1986.
21. Ghosal, D. et al.: Nature, (London), 275, 611-617, 1978.
22. Maniatis, T. et al.: Molecular Cloning, A Laboratory Manual (Cold Spring Harbor Lab., N.Y.), 1982.
23. Selinger, J. et al.: Chem. Scr., 26 , 569-577, 1986.
24. Saiki, R. K. et al.: Science, 230, 1350-1354, 1985.
25. Sanger, F. et al.: Proc. Natl. Acad. Sci., USA, 74 , 5463-5467, 1977.
26. Hsashi, Y. et al.: Eur. J. Immunol., 19 , 1399-1403, 1989.

REFERENCE TO THE DEPOSITION OF MICROORGANISMS

1) The clone (bacteriophage, clone HUD-3) described below was deposited at NCIMB (National Collection of Industrial and Marine Bacteria, P.O. Box 31, 135 Abbey Road, Aberdeen AB9 8DG, Scotland, UK) under the Budapest Treaty, and the deposition date and number are as follows: Deposition date: February 27, 1989 Deposition number: 40122

2) Escherichia coli MV1184 strains carrying the plasmids described below were deposited at the Fermentation Research Institute, Agency of Industrial Science and Technology (1-3 Higashi 1-chome, Tsukuba-shi, Ibaraki Prefecture, 305 Japan) under the Budapest Treaty on December 15, 1988 and the deposition numbers are as follows:

E. coli pPDXP (carrying plasmid pPDXP) FERM BP-2193
E. coli pDA (carrying plasmid pDA) FERM BP-2188
E. coli pDK (carrying plasmid pDK) FERM BP-2189
E. coli pDM (carrying plasmid pDM) FERM BP-2191
E. coli pDN (carrying plasmid pDN) FERM BP-2192
E. coli pDLR (carryng plasmid pDLR) FERM BP-2190

## Claims

**1.** A DNA fragment which at least contains one or more of $D_H$ genes belonging to human immunoglobulin $D_H$ gene families $D_A$, $D_K$ and DIR.

**2.** A DNA fragment consisting of 15 kb, which contains $D_{XP4}$ gene, $D_{A4}$ gene, $D_{K4}$ gene, $D_{N4}$ gene, $DIR_1$ gene, $D_{M1}$ gene, $D_{LR1}$ gene, $D_{XP1}$ gene, $D_{xp'1}$ gene, $D_{A1}$ gene, $D_{K1}$ gene, $D_{N1}$ gene, $DIR_2$ and $D_{M2}$ gene.

**3.** A lambda phage clone which contains the DNA fragment as set forth in Claim 2.

**4.** A recombinant DNA which contains $D_A$, $D_N$, $D_{LR}$, $D_{XP}$, $D_K$ or $D_M$ gene family.

**5.** The recombinant DNA as set forth in Claim 4, in which $D_H$ gene belonging to said gene family is cloned in a plasmid.

**6.** An Escherichia coli strain which carries the recombinant DNA as set forth in Claim 5.

**7.** The recombinant DNA as set forth in Claim 6, in which said $D_H$ gene is $D_{A4}$, $D_{N4}$, $D_{LR1}$, $D_{XP1}$, $D_{K1}$ or $D_{M2}$ gene.

**8.** The E. coli strain as set forth in Claim 5, in which said $D_H$ gene is $D_{A4}$, $D_{N4}$, $D_{LR1}$, $D_{XP1}$, $D_{K1}$ or $D_{M2}$ gene.

**9.** In a method of diagnosing lymphoid neoplasm, which comprises a process to detect lymphoid neoplasm cells, a method of diagnosing lymphoid neoplasm, in which said lymphoid neoplasm cells are detected using a DNA sequence containing $V_H$-$D_H$-$J_H$ joining region characteristic to a patient to be diagnose as a marker.

**10.** The method of diagnosing lymphoid neoplasm as set forth in Claim 9, in which said lymphoid neoplasm is related to B cells.

**11.** The method of diagnosing lymphoid neoplasm as set forth in Claim 9, in which said lymphoid neoplasm is related to T cells.

**12.** The method of diagnosing lymphoid neoplasm, which contains the process to detect lymphoid neoplasm cells, as set forth in Claim 9, 10 or 11, comprising steps of
a) selecting a DNA sequence conserved in $V_H$ in high frequency from $V_H$-$D_H$.$J_H$ joining region of DNA of lymphoid neoplasm cells to prepare an oligonucleotide containing said DNA sequence;
b) selecting a DNA sequence conserved in $J_H$ in high frequency from the above-mentioned $V_H$-$D_H$.$J_H$ joining region to prepare an oligonucleotide having a sequence complementary to said DNA sequence;
c) reacting these primers with DNA isolated from a patient to be diagnosed, using the oligonucleotides obtained in the above-mentioned steps a) and b) as the first and second primers, respectively, and thus amplifying DNA;
d) identifying a DNA sequence of the DNA fragment replicated in step (c) to make the identified DNA sequence a diagnostic marker;
e) preparing the oligonucleotide having the DNA sequence selected from $V_H$-$D_H$ junction region of the DNA sequence of the DNA fragment amplified in step c);
f) preparing the oligonucleotide having the DNA sequence complementary to the DNA sequence selected from $D_H$-$J_H$ junction region of the DNA sequence of the DNA fragment amplified in step c);
g) reacting these primers with DNA isolated from a patient to be diagnosed, after treatment, using the oligonucleotides obtained in the above-mentioned steps a) and b) as the third and fourth primers, respectively, to replicate DNA; and
h) judging the presence of DNAs derived from tumor cells in the DNA isolated in step g) from the patient to be diagnosed by detecting the presence of the DNA fragment replicated in step g).

Fig. 1

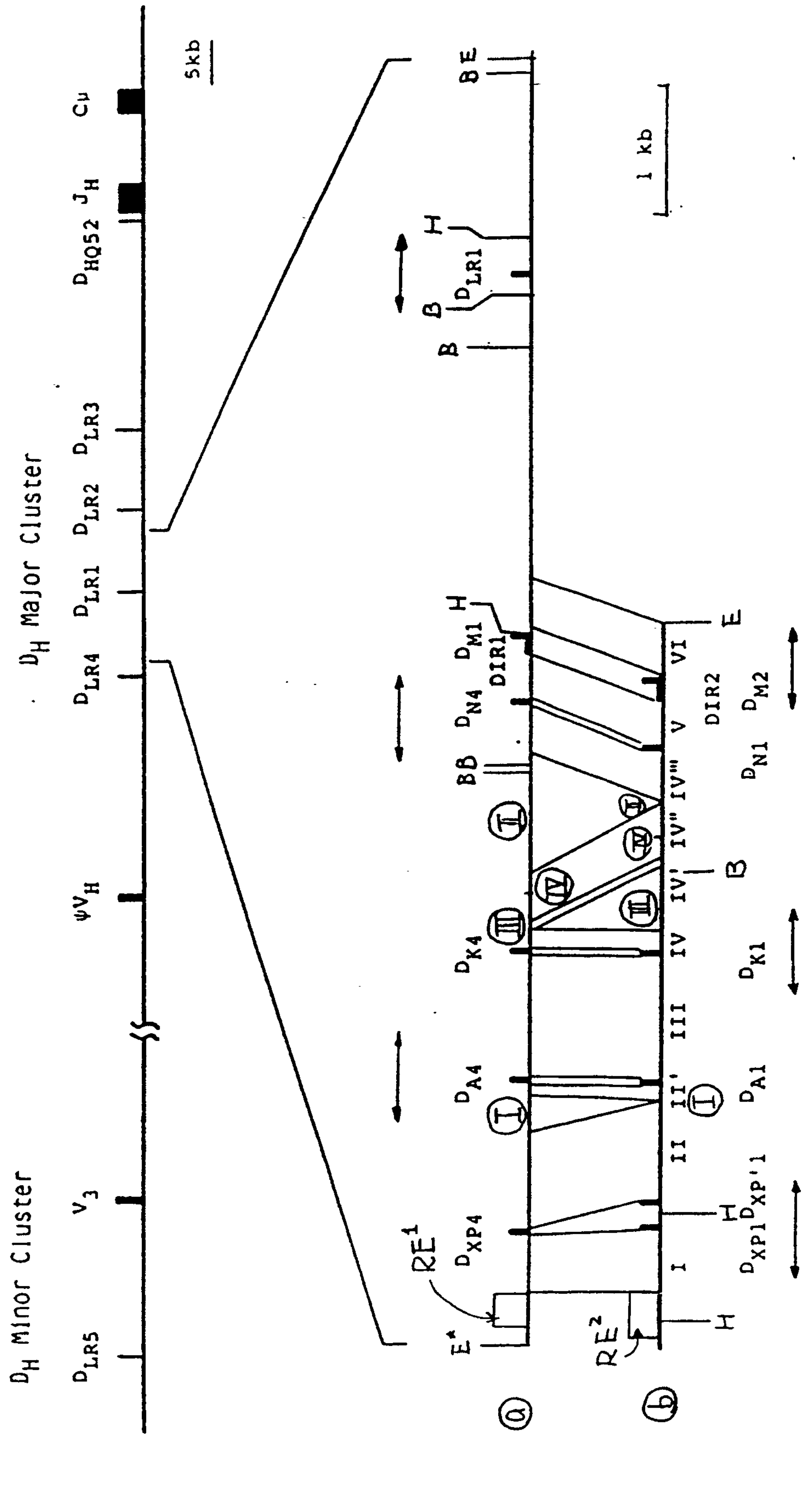
DH Minor Cluster
DH Major Cluster
DLR5
V3
ψVH
DLR4
DLR1
DLR2
DLR3
DHQ52
JH
Cμ
5kb
1 kb
RE1
RE2
DXP4
DA4
DK4
DN4
DM1
DIR1
DIR2
DM2
DN1
DK1
DA1
DXP1
DXP'1
I
II
II'
III
IV
IV'
IV''
IV'''
V
VI
a
b

Fig. 2(a)

```
GAATTCATGCCCCCATCTGGCAGGCACAGAGCATGGCTGGGAGGAGGGGCAGGGACACCAGGCAGGTTGGCACCAACTGAAAATTACAGAAGTCTCATACATCTACCTCAGCCTTGCCT  120
GACCTGGGCCTCACCTGACCTGGACCTCACCTGGCCTGGACCTCACCTGGCCTAGACCTCACCTCTGGGCTTCACCTGAGCTCGGCCTCACCTGACTTGGACCTTGCCTGTCCTGAGCTC  240
ACATGATCTGGGCCTCACCTGACCTGGTTTCACCTGACCTGGGCTTCACCTGACCTGGGCCTCATCTGACCTGGGCCTCACTGGCCTGGACCTCACCTGGCCTGGGCTTCACCTGGCCTC  360
AGGCCTCATCTGCACCTGCTCCAGGTCTTGCTGGAACCTCAGTAGCACTGAGGCTGCAGGGGCTCATCCAGGGTTGCAGAATGACTCTAGAACCTCCCACATCTCAGCTTTCTGGGTGGA  480
GGCACTGGTGGCCCAGGGAATATAAAAAGCCTGAATGATGCCTGCGTGATTTGGGGCAATTTATAAACCCAAAATGGACATGGCCATGCAGCGGGTAGGGACAATACAGACAGATATCA  600
GCCTGAAATGGAGCCTCAGGGCACAGTGGGCACGGACACTGTCCACCTAAGCCAGGGGCAGACCCGAGTGTCCCCGCAGTAGACCTGAGAGCGCTGGGCCCACAGCCTCCCCTCGGTGCC  720
CTGCTACCTCCTCAGGTCAGCCCTGGACATCCCGGGTTTCCCCAGGCTGGCGGTAGGTTTGGGGTGAGGTCTGTGTCACTGTGGTATTACGATTTTTGGAGTGGTTATTATACCCACAGT  840
                                                                                                  D_XP4
GTCACAGAGTCCATCAAAAACCCATCCCTGGGAACCTTCTGCCACAGCCCTCCTGTGGGGCACCGCCGCGTGCCATGTTAGGATTTTGACTGAGGACACAGCACCATGGGTATGGTGCTA  960
CCGCAGCAGTGCAGCCTGTGACCCAAACACACAGGGCAGCAGGCACAACAGACAAGCCCACAAGTGACCACCCTGAGCTCCTGCCTGCCAGCCCTGGAGACCATGAAACAGATGGCCAGG  1080
ATTATCCCATAGGTCAGCCAGACCTCAGTCCAACAGGTCTGCATCGCTGCTGCCCTCCAATACCAGTCCGGATGGGACAGGGCCGGCCCACATTACCATTTGCTGCCATCCGGCCAACA  1200
GTCCCAGAAGCCCCCTCCCTCAAGGCTGGGCCACATGTGTGGACCCTGAGAGCCCCCCATGTCTGAGTAGGGGCACCAGGAAGTGGGCTGGCCCTGTGCACTGTCACTGCCCCTGTGGTCC  1320
CTGGCCTGCCTGGCCCTGACACCTGGGCCTCTCCTGGGTCATTTCCAAGACAGAAGACATTCCCAGGACAGCTGGAGCTGGGAGTCCATCATCCTGCCTGGCCATCCTGAGTCCTGCGCC  1440
TTTCCAAACCTCACCCGGGAAGCCAACAGAGGAATCACCTCCCACAGGCAGAGACAAAGACCTTCCAGAAATCTCTGTCTCTCTCCCCAGTGGGCACCCTCTTCCAGGGCAGTCCTCAGT  1560
GATATCACAGTGGGAACCCACATCTGGATCGGGACTGCCCCCAGAACACAAGATGGCCCACAGGGACAGCCCCACAGCCCAGCCCTTCCCAGACCCCTAAAAGGCGTCCCACCCCCTGCA  1680
(I) TCTGCCCCAGGGCTCAAACTCCAGGAGGACTGACTCCTGCACACCCTCCTGCCAGACATCACCTCAGCCCCTCCTGGAAGGGACAGGAGCGCGCAAGGGTGAGTCAGACCCTCCTGCCCT  1800
CGATGGCAGGCGGAGAAGATTCAGAAAGGTCTGAGATCCCCAGGACGCAGCACCACTGTCAATGGGGGCCCCAGACGCCTGGACCAGGGCCTGCGTGGGAAAGGCCTCTGGGCACACTCA  1920
GGGGCTTTTTGTGAAGGGTCCTCCTACTGTGTGACTACAGTAACTACCACAGTGATGAACCCAGCAGCAAAAACTGACCGGACTCCCAAGGTTTATGCACACTTCTCGCTCAGAGCTCTC  2040
                                      D_A4
CAGGATCAGAAGAGCCGGGCCCAAGGGTTTCTGCCCAGACCCTCGGCCTCTAGGGACATCTTGGCCATGACAGCCCATGGGCTGTGCCCCACACATCGTCTGCCTTCAAACAAGGGCTTC  2160
ACAGGGCTCTGAGGTGACCTCACTGATGACCACAGGTGCCCTGGCCCCTTCCCCGCCAGCTGCACCAGACCCCGTCCTGACAGATGCCCCGATTCCAACAGCCAATTCCTGGGGCCAGGA  2280
ATCGCTGTAGACACCAGCCTCCTTCCAACACCTCTTGCCAATTGCCTGGATTCCCATCCCGGTTGGAATCAAGAGGACAGCATCCCCCAGGCTCCCAACAGGCAGGACTCCCACACCCTC  2400
CTCTGAGAGGCCGCTGTGTTCCGTAGGGCCAGGCTGCAGACAGTCCCCCTCACCTGCCACTAGACAAATGCCTGCTGTAGATGTCCCCACCTGGAAAAGACCACTCATGGAGCCCCCAGC  2520
CCCAGGTACAGCCATAGAGAGAGTCTCTGAGGCCCCTAAGAAGTAGCCATGCCCAGTTCTGCCGGGACCCTCGGCCAGGCTGACAGGAGTGGACGCTGGAGCTGGGCCCACACTGGGCCA  2640
```

Fig. 2(b)

```
CATAGGAGCTCACCAGTGAGGGCAGGAGAGCACATGCCGGGGAGCACCCAGCCTCCTGCTGACCAGAGGCCCGTCCCAGAGCCCAGGAGGCTGCAGAGGCCTCTCCAGGGGGACACTGTG 2760
CATGTCTGGTCCCTGAGCAGCCCCCCATGTCCCCAGTCCTGGGGGGCCCCCTGGCACAGCTGTCTGGACCCTCTCTATTCCCTGGGAAGCTCCTCCTGACAGCCCCGCCTCCAGTTCCAG 2880
GTGTGGTTATTGTCAGGGGGTGTCAGACTGTGGTGGATACAGCTATGGTTACCACAGTGGTGCTGCCCATAGCAGCAACCAGGCCAAGTAGACAGGCCCCTGTGCGCAGCCCCAGGCATC 3000
                                 DK4
CACTTCACCTGCTTCTCCTGGGGCTCTCAAGGCTGCTGTTTTCTGCACTCTCCCCTCTGTGGGGAGGGTTCCCTCAGTGGGAGGATCTGTTCTCAACATCCCAGGGCCTCATTCCTGCAA 3120
                                                                                         III
GGAAGGCCAATGATGGGCAACCTCACATGCCGCGGCTAAGATAGGGTGGGCAGCTGGCGGGGACAGGACATCCTGCTGGGGTATCTGTCACTGTGCCTAGTGGGGCACTGGCTCCCAAAC 3240
                    IV
AACGCAGTCCTCGCCAAAATCCCCACGGCCTCCCCCGCTAGGGGCTGGCCTGATCTCCTGCAGTCCTAGGAGGCTGCTGACCTCCAGAATGGCTCCGTCCCCAGTTCCAGGGCGAGAGCA 3360
GATCCCAGGCCGGCTGCAGACTGGGAGGCCACCCCCTCCTTCCCAGGGTTCACTGCAGGTGACCAGGGCAGGAAATGGCCTGAACACAGGGATAACCGGGCCATCCCCAACAGAGTCCA 3480
CCCCCTCCTGCTCTGTACCCCGCACCCCCAAGGCCAGCCCATGACATCCGACAACCCCACACCAGAGTCACTGCCCGGTGCTGCCCTAGGGAGGACCCCTCAGCCCCCACCCTGTCTAGA 3600
GGACTGGGGAGGACAGGACACGCCCTCTCCTTATGGTTCCCCCACCTGGCTCTGGCTGGGACCCTTGGGGTGTGGACAGAAAGGACGCTTGCCTGATTGGCCCCCAGGAGCCCAGAACTT 3720
CTCTCCAGGGACCCCAGCCCGAGCACCCCCTTACCCAGGACCCAGCCCTGCCCCTCCTCCCATCTGCTCTCCTCTCATCACCCCATGGGAATCCAGAATCCCCAGGAAGCCATCAGGAAG 3840
GGCTGAGGAGGAAGTCGGGCCACGTGCACCACCAGGCAGGAGGCTCCGTCTTTGTGAACCCAGGGAGGTGCCAGCCTCCTAGAGGGTATGGTCCACCCTGCCTATGGCTCCCACAGTGG 3960
(II)
CAGGCTGCAGGGAAGGACCAGGGACGGTGTGGGGAGGGCTCAGGGCCGCGCGGTGCTCCATCTTGGATGAGCCCATCTCTCTCACCCACGGACTCACCCACCTCCTCTCCACCCTGGT 4080
CACACGTCGTCCACACCATCCTAAGTCCCACCTACACCAGAGCCGGCACAGCCAGTGCAGACAGAGGCTGGGGTGCAGGGGGGCCGCCAGGGCAGCTTTGGGGAGGGAGGAATGGAGGAA 4200
GGGGAGTTCAGTGAAGAGGCCCCCCTCCCCTGGGTCCAGGATCCTCCTCTGGGACCCCCGGATCCCATCCCCTCCAGGCTCTGGGAGGAGAAGCAGGATGGGAGAATCTGTGCGGGACCC 4320
TCTCACAGTGGAATACCTCCACAGCGGCTCAGGCAAGACCCAAAAGCCCCTCAGTGAGCCCTCCACTGCAGTCCTGGGCCTGGGTAGCAGCCCCTCCCACAGAGGATGAACCCAGCACCC 4440
CGAGGATGTCCTGCCAGGGGGAGCTCAGAGCCATGAAGGAGCAGGATATGGACCCCCGATACAGGCACAGACCTCAGCTCCATTCAGGACTGCCACGTCCTGCCCTGGGAGGAACCCCT 4560
TTCTCTAGTCCCTGCAGGCCAGGAGGCAGCTGACTCCTGACTTGGACGCCTATTCCAGACACCAGACAGAGGGGCAGGCCCCCCAGAACCAGGGATGAGGACGCCCCGTCAAGGCCAGAA 4680
AAGACCAAGTTGTGCTGAGCCCAGCAAGGGAAGGTCCCCAAACAAACCAGGAACGTTTCTGAAGGTGTCTGTGTCACAGTGGAGTATAGCAGCTCGTCCCACAGTGACACTCGCCAGGCC 4800
                                                                                             DN4
AGAAACCCCATCCCAAGTCAGCGGAATGCAGAGAGAGCAGGGAGGACATGTTTAGGATCTGAGGCCGCACCTGACACCCAGGCCAGCAGACGTCTCCTGTCCATGGCACCCTGCCATGTC 4920
CTGCATTTCTGGAAGAACAAGGGCAGGCTGAAGGGGGTCCAGGACCAGGAGATGGGTCCCCTCTACCCAGAGAAGGAGCCAGGCAGGACACAAGCCCCCTCCCCATTGAGGCTGACCTGC 5040
CCAGACGGTCCTGGGCCCACCCCACACACCGGGGCGGAATGTGTGCAGGCCTCGGTCTCTGTGGGTGTTCCGCTAGCTGGGGCTCACAGTGCTCACCCCACACCTAAAACGAGCCACAGC 5160
CTCAGAGCCCCTGAAGGAGACCCCGCCCACAAGCCCAGCCCCCACCCAGGAGGCCCCAGAGCACAGGGCGCCCCGTCGGATTCTGAACAGCCCCGAGTCACAGTGGGTATAACTGGAACT 5280
                                                                                                                  DM1
```

Fig. 2(c)

ACCACTGTGAGAAAAGCTTCGTCCAAAACGGTCTCCTGGCCACAGTCGGAGGCCCCGCCAGAGAGGGGAGCAGCCACCCCAAACCCATGTTCTGCCGGCTCCCATGACCCCGTGCACCTG 5400

GAGCCCCACAGTGTCCCCACTGGATGGGAGGACAAGGGCCGGGGGCTCCGGCGGGTCGGGGCAGGGGCTTGATGGCTTCCTTCTGCCGTGGCTCCAGTGCCCCTGGCTGGAGTTGACCCT 5520

TCTGACAAGTGTCCTCAGAGAGTCAGGGATCAGTGGCACCTCCCAACATCAACCCCACGCAGCCCAGGCACAAACCCCACATCCAGGGCCAACTCCAGGAACAGAGACACCCCAATACCC 5640

TGGGGGACCCCAACCCTGATGACTCCCGTCCCATCTCTGTCCCTCACTTGGGGCCTGCTGCGGGGGCGAGCACTTGGGAGCAAACTCAGGCTTAGGGGACACCACTGTGGGCCTGACCTCG 5760

AGCAGGCCACAGACCCTTCCCTCCTGCCCTGGTGCAGCACAGACTTTGGGGTCTGGGCAGGGAGGAACTTCTGGCAGGTCACCAAGCACAGAGCCCCCAGGCTGAGGTGGCCCCAGGGGG 5880

AACCCCAGCAGGTGGCCCACTACCCTTCCTCCCAGCTGGACCCCATGTCTTCCCCAAGATAGGGGTGCCATCCAAGGCAGGTCCTCCATGGAGCCCCCTTCAGGCTCCTCTCCAGACCCC 6000

ACTGGGCCTCAGTCCCCACTCTAGGAATGCAGCCACCACGGGCACACCAGGCAGCCCAGGCCCAGCCACCCTGCAGTGCCCAAGCCCACACCCTGGAGGAGAGCAGGGTGCGTCTGGGAG 6120

GGGCTGGGCTCCCCACCCCCACCCCCACCTGCACACCCCACCCACCCTTGCCCGGGCCCCCTGCAGGAGGGTCAGAGCCCCCATGGATATGGACTTAGGGTCTCACTCACGCACCTCCC 6240

CTCCTGGGAGAAGGGGTCTCATGCCCAGATCCCCCCAGCAGCGCTGGTCACAGGTAGAGGCAGTGGCCCCAGGGCCACCCTGACCTGGCCCCTCAGGCATCCTCTAGCCCTGGCTGCCCT 6360

GCTGTCCCTGGGAGGCCTGGGCTCCACCAGACCACAGGTCTAGGGCACCGCCCACACTGGGGCCGCCCACACACAGCTCACAGGAAGAAGATAAGCTCCAGACCCCCAGGCCCGGGACCT 6480

GCCTTGCTGCTACGACTTCCTGCCCCAGACCTCGTTGCCCTCCCCCGTCCACTTACACACAGGCCAGGAAGACTGTTCCCACACAGACCAACCCCAGACGGGGACCACCTGGCACTCAGG 6600

TCACTGCCATTTCCTTCTCCATTCACTTCCAATGCCTCTGTGCTTCCTCCTCCTCCTTCCTTCGGGGGAGCACCCTGTGCAGCTCCTCCCTGCAGTCCACACCCTGGGGAGACCCGACCC 6720

TGCAGCCCACACCCTGGGGAGACCTGACCCTCCTCCAGCCCTTTCTCCCCCGCTGCTCTTGCCACCCACCAAGACAGCCCTGGGGTCCTGTCCCTACAGCCCCCACCCAGTTCTCTACCT 6840

AGACCCGTCTTCCTCCCTCTAAACACCTCTCCCAGGCCAACCCTACACCTGCAGGCCCTCCCCTCCACTGCCAAAGACCCTCAGTTTCTCCTGCCTGTGCCCACCCCCGTGCTCCTCCTG 6960

CCCACAGCTCGAGCTCTTCCTCTCCTAGGGCCCCTGAGGGATGGCATTGACCGTGCCCTCGCACCCACACACTGCCCATGCCCTCACATTCCTCCTGGCCACTCCAGCCCCACTCCCCTC 7080

TCAGGCCTGGCTCTGGTATTTCTGGGACAAAGCCTTACCCAAGTCTTTCCCATGCAGGCCTGGCCCCTTACCCTCACTGCCCGGTTACAGGGCAGCCTCCTGTGCACAGAAGCAGGGAGC 7200

TCAGCCCTTCCACAGGCAGAAGGCACTGAAAGAAATCGGCCTCCAGCGCCTTGACACACGTCTGCCTGTGTCTCTCACTGCCCGCACCTGCAGGGAGGCTCGGCACTCCCTCTAAAGACG 7320

AGGGATCCAGGCAGCAGCATCACAGGAGAATGCAGGGCTACCAGACATCCCAGTCCTCTCACAGGCCTCTCCTGGGAAGAGACCTGAAGACGCCCAGTCAACGGAGTCTAACACCAAACC 7440

TCCCTGGAGGCCGATGGGTAGTAACGGAGTCATTGCCAGACCTGGAGGCAGGGGAGCAGTGAGCCCGAGCCCACACCATAGGGCCAGAGGACAGCCACTGACATCCCAAGCCACTCACTG 7560

GTGGTCCCACAACACCCCATGGAAAGAGGACAGACCCACAGTCCCACCTGGACCAGGGCAGAGACTGCTGAGACCCAGCACCAGAACCAACCAAGAAACACCAGGCAACAGCATCAGAGG 7680

GGGCTCTGGCAGAACAGAGGAGGGGAGGTCTCCTTCACCAGCAGGCGCTTCCCTTGACCGAAGACAGGATCCATGCAACTCCCCCAGGACAAAGGAGGAGCCCCTTGTTCAGCACTGGGC 7800

Fig. 2(d)

TCAGAGTCCTCTCCAAGACACCCAGAGTTTCAGACAAAAACCCCCTGGAATGCACAGTCTCAGCAGGAGAGCCAGCCAGAGCCAGCAAGATGGGGCTCAGTGACACCCGCAGGGACAGGA 7920
GGATTTTGTGGGGGCTCGTGTCACTGTGAGGATATTGTACTAATGGTGTATGCTATACCCACAGTGACACAGCCCCATTCCCAAAGCCCTACTGCAAACGCATTCCACTTCTGGGGCTGA 8040
D LR1
GGGGCTGGGGGAGCGTCTGGGAAATAGGGCTCAGGGGTGTCCATCAATGCCCAAAACGCACCAGACTCCCCTCCATACATCACACCCACCAGCCAGCGAGCAGAGTAAACAGAAAATGAG 8160
AAGCAAGCTGGGGAAGCTTGCACAGGCCCCAAGGAAAGAGCTTTGGCGGGTGTGTAAGAGGGGATGCGGGCAGAGCCTGAGCAGGGCCTTTTGCTGTTTCTGCTTTCCTGTGCAGAGAGT 8280
TCCATAAACTGGTGTTCGAGATCAATGGCTGGGAGTGAGCCCAGGAGGACAGCGTGGGAAGAGCACAGGGAAGGAGGAGCAGCCGCTATCCTACACTGTCATCTTTCGAAAGTTTGCCTT 8400
GTGCCCACACTGCTGCATCATGGGATGCTTAACAGCTGATGTAGACACAGCTAAAGAGAGAATCAGTGAGATGGATTTGCAGCACAGATCTGAATAAATTCTCCAGAATGTGGAGCAGCA 8520
CAGAAGCAAGCACACAGAAAGTGCCTGATGCAAGGACAAAGTTCAGTGGGCACCTTCAGGCATTGCTGCTGGGCACAGACACTCTGAAAAGCCCTGGCAGGAACTCCCTGTGACAAAGCA 8640
GAACCCTCAGGCAATGCCAGCCCCAGAGCCCTCCCTGAGAGCCTCATGGGCAAAGATGTGCACAACAGGTGTTTCTCATAGCCCCAAACTGAGAGCAAAGCAAACGTCCATCTGAAGGAG 8760
AACAGGCAAATAAACGATGGCAGGTTCATGAAATGCAAACCCAGACAGCCACAAGCACAAAAGTACAGGGTTATAAGCGACTCTGGTTGAGTTCATGACAATGCTGAGTAATTGGAGTAA 8880
CAAAGTAAACTCCAAAAAATACTTTCAATGTGATTTCTTCTAAATAAAATTTACACCCTGCAAAATGAACTGTCTTCTTAAGGGATACATTTCCCAGTTAGAAAACCATAAAGAAAACCA 9000
AGAAAAGGATGATCACATAAACACAGTGGTGGTTACTTCTGCTGGGGAAGGAAGAGGGTATGAACTGAGATACACAGGGTGGGCAAGTCTCCTAACAAGAACAGAACGAATACATTACAG 9120
TACCTTGAAAACAGCAGTTAAACTTCTAAATTGCAAGAAGAGGAAAATGGACACAGTTGTGTTTAGAAAATTCTCAGTCCAGCACTGTTCATAATAGCAAAGACATTAACCCAGGTCGGA 9240
TAAATAAGCGATGACACAGGCAATTGCACAATGATACAGACATATATTTAGTATATGAGACATCGATGATGTATCCCCAAATAAACGACTTTAAAGAGATAAAGGGCTGATGTGTGGTGG 9360
CATTCACCTCCCTGGGATCCCCGGACAGGTTGCAGGCTCACTGTGCAGCAGGGCAGGCGGGTACCTGCTGGCAGTTCCTGGGGCCTGATGTGGAGCAAGCGCAGGGCCATATATCCCGGA 9480
GACGGCACAGTCAGTGAATTCCAGAGAGAAGCAACTCAGCCACACTCCCCAGGCAGAGCCCGAGAGGGACGCCCACGCACAGGGAGGCAGAGCCCAGCACCTCCGCAGCCAGCACCACCT 9600
GTGCACGGGCCACCACCTTGCAGGCACAGAGTGGGTGCTGAGAGGAGGGGCAGGGACACCAGGCAGGTGAGCACCCAGAGAAAACTGCAGACGCCTCACACATCCACCTCAGCCTCCCCT 9720
GACCTGGACCTCACTGCCTGGGCCTCACTTAACCTGGGCTTCACCTGACCTTGGCCTCACCTGACTTGGACCTGCCTGTCCCAAGCTTTACCTGACCTGGGCCTCAACTCACCTGAACGT 9840
CTCCTGACCTGGGTTTAACCTGTCCTGGAACTCACCTGGCCTTGGCTTCCCCTGACCTGGACCTCATCTGGCCTGGGCTTCACCTGGCCTGGGCCTCACCTGACCTGGACCTCATCTGGC 9960
CTGGACCTCACCTGGCCTGGACTTCACCTGGCCTGGGCTTCACCTGACCTGGACCTCACCTGGCCTCGGGCCTCACCTGCACCTGCTCCAGGTCTTGCTGGAGCCTGAGTAGCACTGAGG 10080
GTGCAGAAGCTCATCCAGGGTTGGGGAATGACTCTAGAAGTCTCCCACATCTGACCTTTCTGGGTGGAGGCAGCTGGTGGCCCTGGGAATATAAAAATCTCCAGAATGATGACTCTGTGA 10200
TTTGTGGGCAACTTATGAACCCGAAAGGACATGGCCATGGGGTGGGTAGGGACATAGGGACAGATGCCAGCCTGAGGTGGAGCCTCAGGACACAGGTGGCACGGACACTATCCACATAA 10320
GCGAGGGATAGACCCGAGTGTCCCCACAGCAGACCTGAGAGCGCTGGGCCCACAGCCTCCCCTCAGAGCCCTGCTGCCTCCTCCGGTCAGCCCTGGACATCCCAGGTTTCCCCAGGCCTG 10440
CCGGTAGGTTTAGAATGAGGTCTGTGTCACTGTGGTATTACGATATTTTGACTGGTTATTATAACCACAGTGTCACAGAGTCCATCAAAAACCCATGCCTGGAAGCTTCCCGCCACAGCC 10560
D XP1

Fig. 2(e)

```
CTCCCCATGGGGCCCTGCTGCCTCCTCAGGTCAGCCCCGGACATCCCGGGTTTCCCCAGGCTGGGCGGTAGGTTTGGGGTGAGGTCTGTGTCACTGTGGTATTACTATGGTTCGGGGAGT   10680
                                                                                                                 Dχρ'1
TATTATAACCACAGTGTCACAGAGTCCATCAAAAACCCATCCCTGGGAGCCTCCCGCCACAGCCCTCCCTGCAGGGACCGTACGTGCCATGTTAGGATTTTGATCGAGGAGACAGCACCA   10800
TGGGTATGGTGGCTACCACAGCAGTGCAGCCTGTGACCCAAACCCACAGGGCAGCAGGCACGATGGACAGGCCCGTGACTGACCACGCTGGGCTCCAGCCTGCCAGCCCTGGAGATCATG   10920
AAACAGATGGCCAAGGTCACCCTACAGGTCATCCAGATCTGGCTCCGAGGGGTCTGCATCGCTGCTGCCCTCCCAACGCCAGTCCAAATGGACAGGGACGGCCTCACAGCACCATCTGC   11040
TGCCATCAGGCCAGCGATCCCAGAAGCCCCTCCCTCAAGGCTGGGCCACATGTGTGGACATGAGAGCCCTCATGTCTGAGTAGGGGCACAGGAGGGAGGGGCTGGCCCTGTGCACTGTCC   11160
CTGCCCCTGTGGTCCCTGGCCTGCCTGGCCCTGACACTGAGCCTCTCCTGGGTCATTTCCAAGACAGAAGACATTCCTGGGGACAGCCGGAGCTGGGCGTCGCTCATCCTGCCCGGCCGT   11280
CCTGAGTCCTGCTCATTTCCAGACCTCACCGGGGAAGCCAACAGAGGACTCGCCTCCCACATTCAGAGACAAAGAACCTTCCAGAAATCCCTGCCTCTCTCCCCAGTGGACACCCTCTTC   11400
CAGGACAGTCCTCAGTGGCATCACAGCGGCCTGAGATCCCCAGGACGCAGCACCGCTGTCAATAGGGGCCCCAAATGCCTGGACCAGGGCCTGCGTGGGAAAGGTCTCTGGCCACACTCG   11520
                               ▲(1)
GGCTTTTTGTGAAGGGCCCTCCTGCTGTGTGACTACAGTAACTACCATAGTGATGAACCCAGTGGCAAAAACTGGCTGGAAACCCAGGGGCTGTGTGCACGCCTCAGCTTGGAGCTCTCC   11640
                                    DA1
AGGAGCACAAGAGCCGGGCCCAAGGATTTGTGCCCAGACCCTCAGCCTCTAGGGACACCTGGGCCATCTCAGCCTGGGCTGGTGCCCTGCACACCATCTTCCTCCAAATAGGGGCTTCAG   11760
AGGGCTCTGAGGTGACCTCACTCATGACCACAGGTGACCTGGCCCTTCCCTGCCAGCTATACCAGACCCTGTCTTGACAGATGCCCCGATTCCAACAGCCAATTCCTGGGACCCTGAATA   11880
GCTGTAGACACCAGCCTCATTCCAGTACCTCCTGCCAATTGCCTGGATTCCCATCCTGGCTGGAATCAAGAAGGCAGCATCCGCCAGGCTCCCAACAGGCAGGACTCCCGGACACCCTCC   12000
TCTGAGAGGCCGCTGTGTTCCGCAGGGCCAGGCCCTGGACAGTTCCCCTCACCTGCCACTAGAGAAACACCTGCCATTGTCGTCCCCACCTGGAAAAGACCACTCGTGGAGCCCCCAGCC   12120
CCAGGTACAGCTGTAGAGAGAGTCCTCGAGGCCCCTAAGAAGGAGCCATGCCCAGTTCTGCCGGGACCCTCGGCCAGGCCGACAGGAGTGGACGCTGGAGCTGGCCCACACTGGGCATAG   12240
GAGCTCACCAGTGAGGGCAGGAGAGCACATGCCGGGGAGCACCCAGCCTCCTGCTGACCAGAGGCCTGCCCCAGAGCCCAGGAGGCTGCAGAGGCCTCTCCAGGGAGACACTGTGCATGT   12360
CTGGTACCTAAGCAGCCCCCCACGTCCCCAGTCCTGGGGGCCCCTGGCTCAGCTGTCTGGACCCTCCCTGTTCCCTGGGAAGCTCCTCCTGACAGCCCCGCCTCCAGTTCCAGGTGTGGT   12480
```

Fig. 2(f)

TATTGTCAGGCGATGTCAGACTGTGGTGGATATAGTGGCTACGATTACCACAGTGGTGCCGCCCATAGCAGCAACCAGGCCAAGTAGACAGGCCCCTGCTGCGCAGCCCCAGGCATCCAC 12600

$D_{K1}$

TTCACCTGCTTCTCCTGGGGCTCTCAAGGCTGCTGTCTGTCCTCTGGCCCTCTGTGGGGAGGGTTCCCTCAGTGGGAGGTCTGTGCTCCAGGGCAGGGATGATTGAGATAGAAATCAAAG 12720

GCTGGCAGGGAAAGGCAGCTTCCCGCCCTGAGAGGTGCAGGCAGCACCACGGAGCCACGGAGTCACAGAGCCACGGAGCCCCCATTGTGGGCATTTGAGAGTGCTGTGCCCCCGGCAGCC 12840

(III)

CAGCCCTGATGGGGAAGCCTGTCCCATCCCACAGCCCGGGTCCCACGGGCAGCGGGCACAGAAGCTGCCAGGTTGTCCTCTATGATCCTCATCCCTCCAGCAGCATCCCCTCCACAGTGG 12960

GGAAACTGAGGCTTGGAGCACCACCCGGCCCCCTGGAAATGAGGCTGTGAGCCCAGACAGTGGGCCCAGAGCACTGTGAGTACCCCGGCAGTACCTGGCTGCAGGGATCAGCCAGAGATG 13080

CCAAACCCTGAGTGACCAGCCTACAGGAGGATCCGGCCCCACCCAGGCCACTCGATTAATGCTCAACCCCCTGCCCTGGAGACCTCTTCCAGTACCACCAGCAGCTCAGCTTCTCAGGGC 13200

CTCATCCCTGCAAGAAGGTCAAGGGCTGGGCCTGCCAGAAACACAGCACCCTCCCTAGCCCTGGCTAAGACAGGGTGGGCAGACGGCTGTGGACGGACATATTGCTGGGGCATTTCTCA 13320

(IV)

CTGTCACTTCTGGGTGGTAGCTCTGACAAAAACGCAGACCCTGCCAAAATCCCCACTGCCTCCCGCTAGGCTGGCCTGGAATCCTGCTGTCCTAGGAGGCTGCTGACCTCCAGGATGGCT 13440

CCGTCCCCAGTTCCAGGGCGAGAGCAGATCCCAGGCAGGCTGTAGGCTGGGAGGCCACCCCTGCCCTTGCCGGGGTTGAATGCAGGTGCCCAAGGCAGGAAATGGCATGAGCACAGGGAT 13560

GACCGGGACATGCCCCACCAGAGTGCGCCCCTTCCTGCTCTGCACCCTGCACCCCCCAGGCCAGCCCACGACGTCCAACAACTGGGCCTGGGTGGCAGCCCCACCCAGACAGGACAGACC 13680

(II)

CAGCACCCTGAGGAGGTCCTGCCAGGGGGAGCTAAGAGCCATGAAGGAGCAAGATATGGGGCCCCCGATACAGGCACAGATGTCAGCTCCATCCAGGACCACCCAGCCCACACCCTGAGA 13800

GGAACGTCTGTCTCCAGCCTCTGCAGGTCGGGAGGCAGCTGACCCCTGACTTGGACCCCTATTCCAGACACCAGACAGAGGCGCAGGCCCCCCAGAACCAGGGTTGAGGACGCCCCGTC 13920

AAAGCCAGACAAAACCAAGGGGTGTTGAGCCCAGCAAGGGAAGGCCCCCAAACAGACCAGGAGGTTTCTGAAGGTGTCTGTGTCACAGTGGGGTATAGCAGCAGCTGGTACCACAGTGAC 14040

$D_{N1}$

ACTCACCCAGCCAGAAACCCCATTCCAAGTCAGCGGAAGCAGAGAGAGCAGGGAGGACACGTTTAGGATCTGAGACTGCACCTGACACCCAGGCCAGCAGACGTCTCCCCTCCAGGGCAC 14160

CCCACCCTGTCCTGCATTTCTGCAAGATCAGGGGCGGCCTGAGGGGGGGTCTAGGGTGAGGAGATGGGTCCCCTGTACACCAAGGAGGAGTTAGGCAGGTCCCGAGCACTCTCCCCATTG 14280

AGGCTGACCTGCCCAGAGAGTCCTGGGCCCACCCCACACACCGGGGCGGAATGTGTGCAGGCCTCGGTCTCTGTGGGTGTTCCGCTAGCTGGGGCTCACAGTGCTCACCCCACACCTAAA 14400

ATGAGCCACAGCCTCCGGAGCCCCCGCAGAGACCCCGCCCACAAGCCCAGCCCCCACCCAGGAGGCCCCAGAGCTCAGGGCGCCCCGTCGGATTCCGAACAGCCCCGAGTCACAGCGGGT 14520

ATAACCGGAACCACCACTGTCAGAATAGCTACGTCAAAAACTGTCCAGTGGCCACTGCCGGAGCCCCGCCAGAGAGGGCAGCAGCCACTCTGATCCCATGTCCTGCCGGCTCCCATGACC 14640

$D_{M2}$

CCCAGCACGCGGAGCCCACAGTGTCCCCACTGGATGGGAGGACAAGAGCTGGGGATTCCGGCGGGTCGGGGCAGGGGCTTGATCGCATCCTTCTGCCGTGGCTCCAGTGCCCCTGGCTGG 14760

AGTTGACCCTTCTGACAAGTGTCCTCAGAGAGACAGGCATCACCGGCGCCTCCCAACATCAACCCCAGGCAGCACAGGCACAAACCCCACATCCAGAGCCAACTCCAGGAGCAGAGACAC 14880

CCCAATACCCTGGGGGACCCCGACCCTGATGACTTCCCACTGGAATTC 14928

Fig. 3

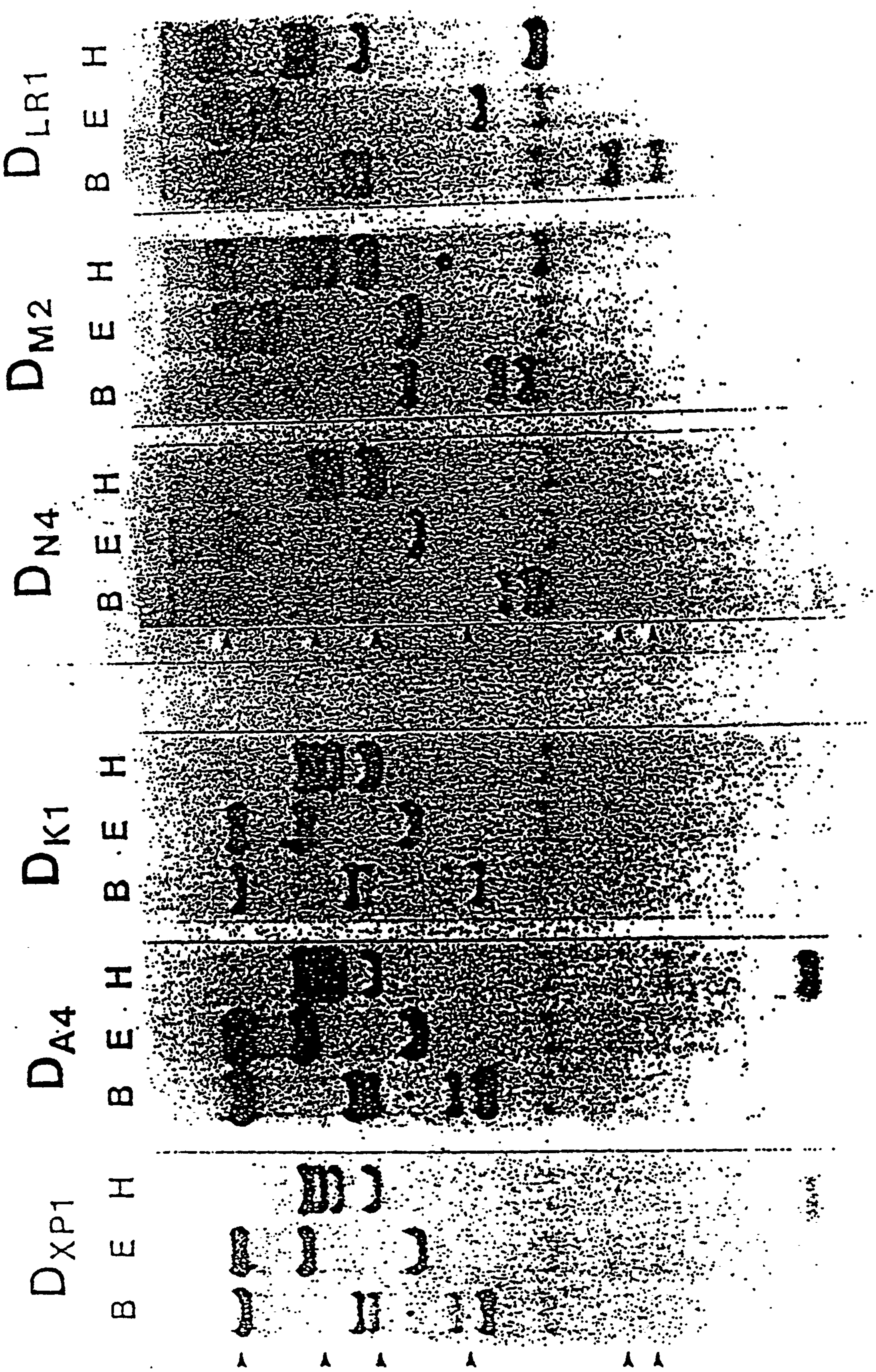

Fig. 4

| | | | | | | | |
|---|---|---|---|---|---|---|---|
| $D_{XP4}$ | GGTTTGGGG | TGAGGTCTGTGT | CACTGTG | GTATTACGATTTTTGGAGTGGTTATTATACC | CACAGTG | TCACAGAGTCCA | TCAAAAACC |
| $D_{XP1}$ | GGTTTAGAA | TGAGGTCTGTGT | CACTGTG | GTATTACGATATTTTGACTGGTTATTATAAC | CACAGTG | TCACAGAGTCCA | TCAAAAACC |
| $D_{XP'1}$ | GGTTTGGGG | TGAGGTCTGTGT | CACTGTG | GTATTACTATGGTTCGGGGAGTTATTATAAC | CACAGTG | TCACAGAGTCCA | TCAAAAACC |
| $D_{A4}$ | GCTTTTTGT | GAAGGGTCCTCC | TACTGTG | TGACTACAGTAACTAC | CACAGTG | ATGAACCCAGCA | GCAAAAACT |
| $D_{A1}$ | GCTTTTTGT | GAAGGGCCCTCC | TGCTGTG | TGACTACAGTAACTAC | CATAGTG | ATGAACCCAGTG | GCAAAAACT |
| $D_{K4}$ | GGTTATTGT | CAGGGGGTGTCA | GACTGTG | GTGGATACAG CTATGGTTAC | CACAGTG | GTGCTGCCCATA | GCAGCAACC |
| $D_{K1}$ | GGTTATTGT | CAGGCGATGTCA | GACTGTG | GTGGATATAGTGGCTACGATTAC | CACAGTG | GTGCCGCCCATA | GCAGCAACC |
| $D_{N4}$ | CGTTTCTGA | AGGTGTCTGTGT | CACAGTG | GAGTATAGCAGC TCGTCC | CACAGTG | ACACTCGCCAGG | CCAGAAACC |
| $D_{N1}$ | GGTTTCTGA | AGGTGTCTGTGT | CACAGTG | GGGTATAGCAGCAGCTGGTAC | CACAGTG | ACACTCACCCAG | CCAGAAACC |
| $D_{M1}$ | GGATTCTGA | ACAGCCCCGAGT | CACAGTG | GGTATAACTGGAACTAC | CACTGTG | AGAAAAGCTTCG | TCCAAAACG |
| $D_{M2}$ | GGATTCCGA | ACAGCCCCGAGT | CACAGCG | GGTATAACCGGAACCAC | CACTGTC | AGAATAGCTACG | TCAAAAACT |
| $D_{LR5}$ | GGATTTTGT | GGGGGCTTGTGT | CACTGTG | AGAATATTGTAATAGTACTACTTTCTATGCC | CACAGTG | ACACAGCCCCAG | TCCCAAAGC |
| $D_{LR4}$ | GGATTTTGT | GGGGGCTCGTGT | CACTGTG | AGGATATTGTAGTAGTACCAGCTGCTATGCC | CACAGTG | ACACAGCCCCAT | TCCCAAAGC |
| $D_{LR1}$ | GGATTTTGT | GGGGGCTCGTGT | CACTGTG | AGGATATTGTACTGGTAATGTATGCTATACC (GG) | CACAGTG | ACACAGCCCCAT | TCCCAAAGC |
| $D_{LR2}$ | GGATTTTGT | GGGGGCTCGTGT | CACTGTG | AGGATATTGTAGTGGTGGTAGCTGCTACTCC | CACAGTG | ACACAGACCCAT | TCCCAAAGC |
| $D_{LR3}$ | GGATTTTGT | GGGGGCTCGTGT | CACTGTG | AGCATATTGTGGTGGTGAT TGCTATTCC | CACAGTG | ACACAACCCCAT | TCCTAAAGC |
| $D_{HQ52}$ | GGTTTTTGG | CTGAGCTGAGAAC | CACTGTG | CTAACTGGGGA | CACAGTG | ATTGGCAGCTCT | ACAAAAACC |

Fig. 5(a)

$\underline{D_{XP}}$ family

| | |
|---|---|
| | *SerAspProPheTrpSerAspTyrTyrAsnPheAspTyrSerTyrThrLeu* |
| 266BL | AAAGTGACCCTTTTTGGAGTGATTATTATAACTTTGACTACTCGTACACTTTG |
| $D_{XP1}$ | GTATTACGATATTTTGACTGGTTATTATAAC |
| | *ArgMetGlnValThrMetValArgGluValMetIleThrSer* |
| CE-1 | CGGATGCAGGTTACTATGGTTCGGGAAGTTATGATAACGTCTA |
| $D_{XP'1}$ | GTATTACTATGGTTCGGGGAGTTATTATAAC |
| | *GlyAlaArgAspValArgTerLeuLeuAlaAlaPhe* |
| CE-114 | CGGAGCGCGTGATGTACGATGACTTCTGGCAGCCTTCC |
| $D_{XP'1}$ | GTATTACTATGG-TTCGGGGAGTTATTATAAC |
| | *ProAsnAlaAspTyrGlyAla* |
| VDJ191 | AACCAAACGCTGACTATGGTGCGA |
| $D_{XP'1}$ | GTATTACTATGGTTCGGGGAGTTATTATAAC |
| | *ArgSerProAspTyrGlyHis* |
| SU-DHL-4 | CGGAGCCCTGATTACGGGCACA |
| $D_{XP'1}$ | GTATTACTATGGTT-CGGGGAGTTATTATAAC |
| | *ArgGlyLeuLeuThrAsnAsn* |
| TS1 | CGAGGCCTTTTGACTAATAATG |
| $D_{XP1}$ | GTATTACGATATTTTGACTGGTTATTATAAC |
| | *GlyTyrAspThrGlyGlyTyrMetAlaArg* |
| TS2 | GGCTATGATACTGGCGGATATATGGCCCGC |
| $D_{XP'1}$ | GTATTACTATGGTTCGGGGAGTTATTATAAC |

Fig. 5(b)

$D_K$ family

```
              GlyGlyTyrSerGlyTyrAspLeuArgProHisAsp
GUV-E3D10     GGTGGATATAGTGGCTACGATTTAGGTCCGCACGACT
              |                           *  |
D_K1           GTGGATATAGTGGCTACGATT-AC

                       LeuAlaThrIleLysAlaProThrLeu
VDJ192                 TTTGGCTACAATTAAAGCGCCCACTTTGCA
                         |        *     |
D_K1          GTGGATATAGTGGCTACGATTAC

              LeuGluGlyArgGlyTyrThrGlyTyrAlaLeuProTyr
WS1           CTGGAGGGGCGTGGATACACTGGCTACGCCCTCCCCTAT
                          |        * *         |
D_K1                      GTGGATATAGTGGCTACGATTAC

              AspValGluLeuArgTyrGlyThrGly
HP1           GATGTGGAGTTGAGGTATGGTACAGGC
                 |       *  **   *     |
D_K4             GTGGA-TACAGCTATGGTTAC
```

$D_{LR}$ family

```
                 IleLeuGlyProTyrSerSerGlyTrpTyrProAsnSerAsp
LR35             GGATCCTGGGGCCGTATAGCAGTGGCTGGTACCCGAACTCGGAC
                              |   * *     *    *|
D_LR2                      AGGATATTGTAGTGG-TGGTAGCTGCTACTCC

                 SerProLeuGlyHisCysSerGlyValArgCysTyrPro
pGG3117          AGTCCCCTCTGGGACATTGTAGTGGTGTTCGGTGCTATCCCC
                          |  *              * * *    |
D_LR2                     AGGATATTGTAGTGGTGGTAGCTGCTACTCC
```

Fig. 6

| | | | |
|---|---|---|---|
| MCE | RPPWRFTGNLGG | WAS | FRQPFVQF |
| NZU | RPPWRFTSDLGSFSP | TEI | VTPAAASLTFSA<br>VVPAA ($D_{LR4}$) |
| POM,LAY | DAGPYVSPTF | BRO | SPVSLVDGWL |
| EU | GYGIYSPEEYN<br>YYYGSGSYYN ($D_{XP',1}$) | GRA | HIYVTL<br>HIVV ($D_{LR3}$) |
| SIE | EWKGQVNVNP | ZAP | TRPGGYFS<br>GYSS ($D_{N1}$) |
| WOL | EYGFDTSD | JON | VVVSTS<br>VVV ($D_{LR2,3}$) |
| NDCL | SDPFWSDYYNFDYSYTL<br>FWSGYY ($D_{XP4}$) | BUT | DLAAARLFGK<br>AAR ($D_{N4}$)<br>AAA ($D_{N1}$) |
| MOT | GAHYSDTDDSGTSLGP<br>SGGS ($D_{LR2}$) | DOB | GYIWNG<br>YNWN ($D_{M1}$) |
| HUS | BRBBYGBF | WEA | GWLLN<br>WLL ($D_{XP4,1}$) |
| COR | ITVIPAPAGYMDV<br>IVVVPA ($D_{LR4}$) | NIE | IRDTAMF<br>DTAM ($D_{K4}$) |
| DAW | SCGSQ | CAM | DRPLYGBYRA<br>RILY ($D_{LR1}$) |
| OU | VVNSVMAG | GAL | GWGGG |
| HE | RHPRTL | TRO | TNNFNWSTFSL |
| NEWM | NLIAGCI | KOL | DGGHGFCSSASCFGP<br>GYCSSTSC ($D_{LR4}$) |
| WAH | GNPPPYYDIGTGSDD<br>YYDILTG ($D_{XP1}$) | HIL | DPDILTAFS<br>DILT ($D_{XP1}$) |
| TUR | LSVTAV | BUR | LIAVAGTR<br>IAAAGT ($D_{N1}$) |
| TIL | GKVSAYY<br>SGYY ($D_{XP4}$) | GA | SGIALGSVAGT<br>GIA ($D_{N1}$) |

Fig. 7(a)

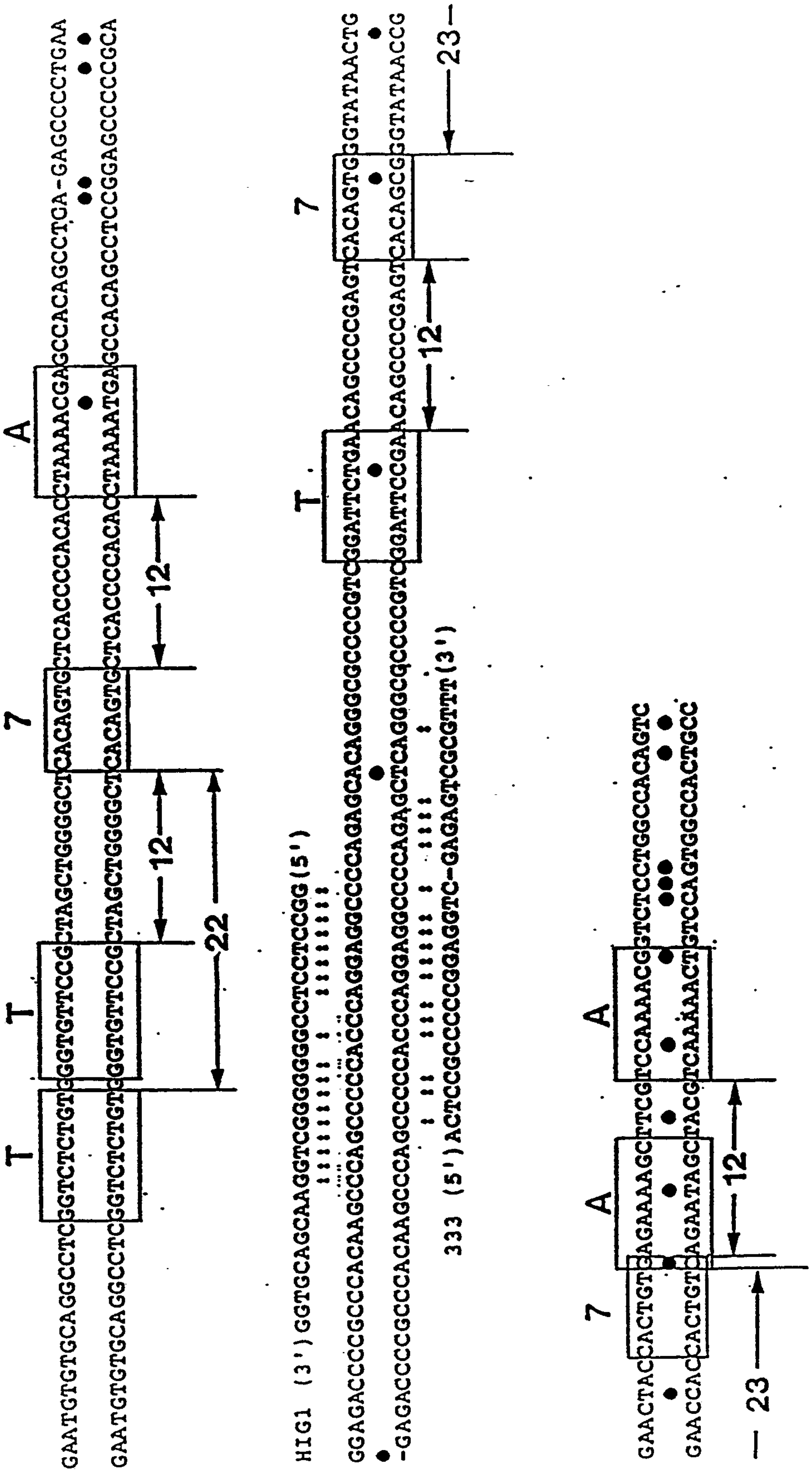

Fig. 7(b)

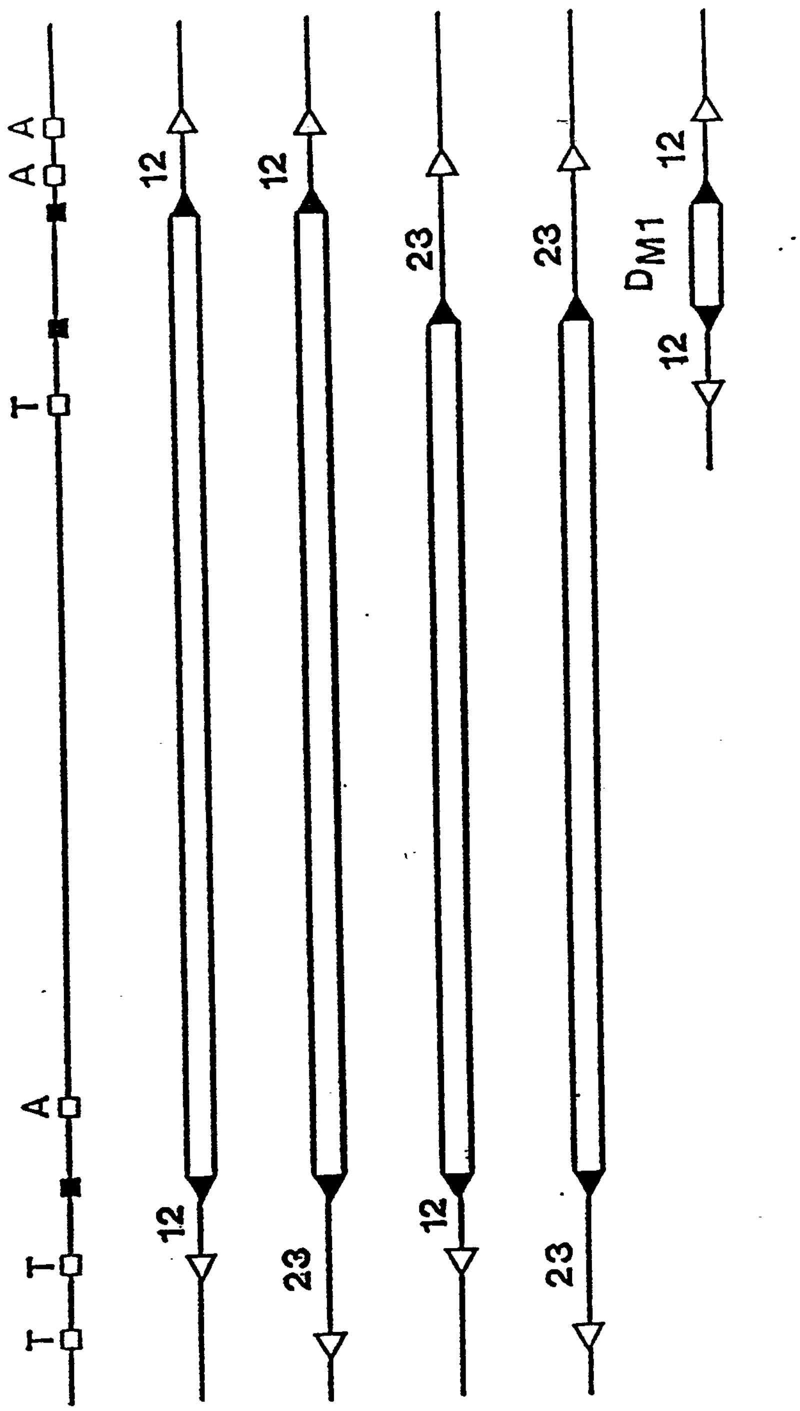

Fig. 8

(a)

$D_{SP2.5}$(mouse) GATTTTTGT CAAGGTATCTAC TACTGTG TCTACTATGGTAACTAC CACAGTG ATATATCCAGCA ACAAAAACC

$D_{A4}$(human) GCTTTTTGT GAAGGGTCCTCC TACTGTG TG-ACTACAGTAACTAC CACAGTG ATGAACCCAGCA GCAAAAACT

(b)

$D_{SP2.2}$(mouse) GGCCCC-TGACACT-CTGCACTGCTACCTCTGGCCCCACCAGGCAATGTTCCTGCAGA-ATCTCTTACCTTACTTGGCAGG---

$D_{K4}$(human) GGCCCCCTGGCACAGCTGT-CTGGACCCTCTCTATTC-CCTGGGAA-GCTCCTCCTGACAGCCCCGCCTCCAGTTC-CAGGTGT

$D_{SP2.2}$(mouse) GATTTTTGT CAAGGGATCTAC TACTGTG ---TCTA---CTATGATTACGAC CACAGTG ATATATCCAGCA ACAAAAACC

$D_{K4}$(human) GGTTATTGT CAGGGGGTGTCA GACTGTG GTGGATACAGCTATGGTTAC--- CACAGTG GTGCTGCCCATA GCAGCAACC

$D_{SP2.2}$(mouse) CAGTATGCCCAGCAGAC--TCTTCA--GCTCAAAA-CAGTGAGTCA--AGAGGAGCTTGCACCTTAGGGGGCT

$D_{K4}$(human) -AG---GCCAAGTAGACAGGCCCCTGTGCGCAGCCCCAGGCATCCACTTCACCTGCTT-CTCCT---GGGGCT

Fig. 9(a-1)

Similarity

```
5'D_XP4   GCAGACCCG-AGTGTCCCCGCAGTAGACCTGAGAG---CGCTGGGCCCACAGC---CT--CCCCTC--GGTGCCCTG-CTACCTCCTCAGGTCAGCCCT
          :::: :::  : :::::::  :::    :::: : :    : ::::    ::::::    ::    :::::    : :::::   : ::::::  : ::::::
5'D_K4    GCAGCCCCCA-TGTCCCC--AGT---CCTGGGGGGCCCCCTGG---CACAGCTGTCTGGACCCTCTCTATTCCCTGGGAAGCTCCTCCTGACAGCCCC

          GGACATCCCGGGTTTCCCCAGGCTGGCGGTAGGTTTGGG
          :  : :::   :::   :::::::    : ::::   :
          G--CCTCC--AGTT---CCAGG-TG----T-GGTTATTG

5'D_XP4   GTGAGGTC-TGTGTCACTGTGGT
           : :::   :::  ::::::::
5'D_K4    -TCAGGGGGTGTCAGACTGTGGT

5'D_A4    AGGACGCAGCACCACTGTCAATGGGGGCCCCAGAC--G-CC---TGGACCAGGGCCT-GCGTGGGAAAGG-CCTCTGGGCACACTCAGGGGCTTTTTGTGAAGG-GTCC---TCCTACTGTG
          :::::::     :: : ::::: ::     :::::   : ::   : :  : : :::  ::  ::: :::: :: :      :: :: ::::  : :::  :::::: :::      ::  :: :::
5'D_N4    AGGACGC----CC-C-GTCAA-GG-----CCAGAAAAGACCAAGTTGTGCTGAGCCCAGCAAGGG-AAGGTCCCCAAA-CAAAC-CAGGAACGTTTC-TGAAGGTGTCTGTGTC--ACAGTG

5'D_XP4   GCTGGG---CCCACAGCCTCCCCTCGGTGCCCTGCTACCTCCTCAGG-TCAGCCCT--GGACATCCCGGGTTTCCCCAGGCTGGCGGTAGG-TTTGGGGTGAGGTCTGTGTCACTGTG
          ::::::    ::: : :   :::  :   ::::::  ::: ::::    :::::::   :   :   :   :        :::: : :  ::  :  :::::  :::: :   :    :::::::
5'D_HQ52  GCTGGGGGTCCC-CTGAA-CCCGACCC-GCCCTGAGACCGCAGCCACATCAGCCCCCAGCCCCACAGGCCCCCTACCAGCC-GCAGG--GTTTTTGGC-TGAGCTGAGAACCACTGTG
```

Fig. 9(a-2)

Complementarity

```
5'D_XP4  CCCCTCGGTGCCCTGC-TACCT--C-CTCAGGTCAG-CCCTGGACAT-CCCGGGTTTCCCCAGGCTG---GCGGTAGG--TTTGGGG-TGAGGTCTGTGTCACTGTGG-TAT
3'D_LR1  GGGGA--CTCGGGATAAAGGGTCTGCGAGGGG-GTCGGGGA---GTCGGGGTCTTCACC-TTACG-CAAACGTCATCCCGAAACCCTTACCCC-GACACAGTGACACCCATA

5'D_M1   GGAGACCCC---GCCCACAAGCCCAG-C-CCCCACCCAGGAG----GCCCCAGA-GCACAGGGCG--CCCCGTC-GGATTCT-GAACAGCCCCGAGTCACAGTGGGTATA
3'D_LR1  CCTGTGGGGACTCGGGATAAAGGGTCTGCGAGG-GGGTCGGGGAGTCGGGGTCTTCACCTTACGCAAACGTCATCCCGAAACCCTTACCCCGACACAGTGACACCCATAT

5'D_N4   AGAACCAGGG--ATGAGGACGCC----CCGTCAAGGCCAGAAAAGACC-AAGTTGTGCTGA-------GCCC---AGCAAGGGAAGGTCCCCA-AACAAA-CCA-GGAAC-
3'D_XP4  TCGTGGTATGGGTA--CCA-CGACACAGG-AGT-CAG---TTTTA-GGATTGTAC-CGTGCGCCGCCACGGGGTGTC-CTCCCG-ACAC-CGTCTTCCAAGGGTCCCTACC

         GTTTCTGAAGGTGTCTGTGTCACAGTG
         CAAAAACTACCTGAGACACTGTGACAC

5'D_LR1  AGA---CA-AAAAC-CC--CCTGGAATGCACAGTCTCAGCAGGAGAGCCAGCCAGA-GCC-A-GCAAGATGGGGCT---CAGTGACACCCG-CAGGGACA-GGA--GGAT
3'D_K4   TCTCACGTCTTTTGTCGTCGGA----AC-TCTC-G-GG-GTCCTCTTCGTCCA-CTTCACCTACGG---ACCCCGACGCGTGTCCCCGGACAGA---TGAACCGGACCAA

         TTTGTGGGGGCTCGTGTCACTGTG
         CGACGATACCCGTCGTGGTGACAC
```

Fig. 9(b)

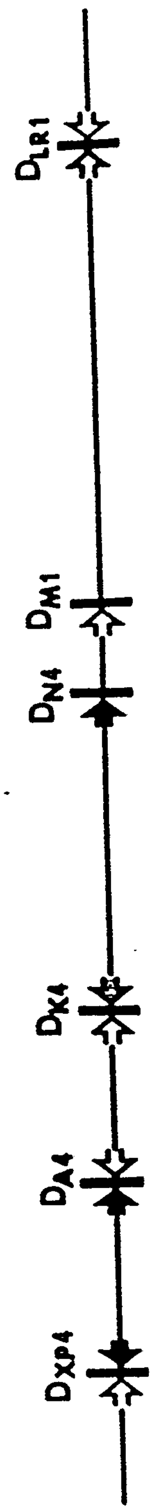

Fig. 10

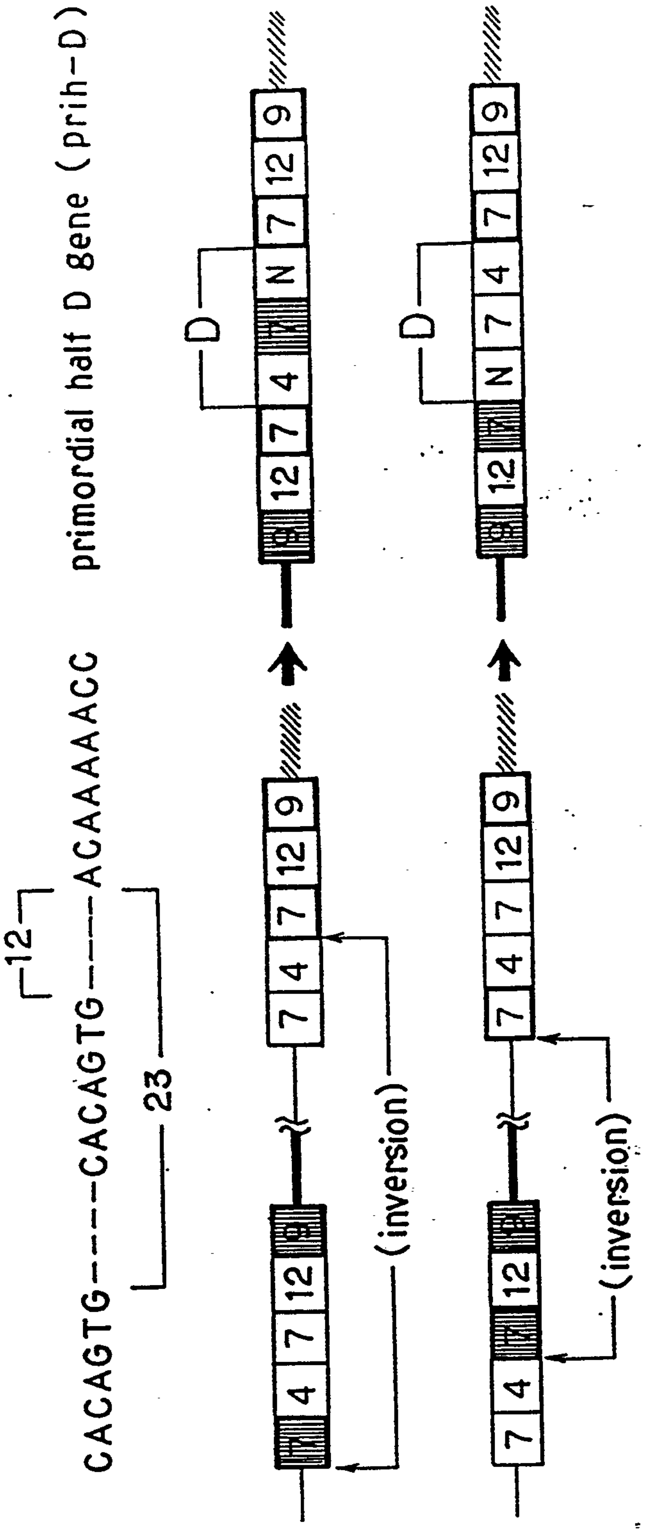
primordial half D gene (prih-D)
CACAGTG----CACAGTG----ACAAAAACC
12
23
D
D
(inversion)
(inversion)

Fig. 11(a)

Similarity

```
3'D_N4      AGTGACACTCGCCAGGCCAGAAACCCCATCCCAAGTCAGCGGAATGCAGAGAGAGCAGG-G-AGGACATG
            ::::  :  : ::::: :: :::::   ::::   :::  :::  :: ::: :  : :: : :::   ::
3'V_71-2    AGTGTGAG-C-CCAGGACACAAACC---TCCC---TCA-TGGA-CGCGGAGGGGACCGGCGCAGGTGCTG

                                         TTTAGGATCTG-AGGC--CGCACCTGACACCCA
                                          : :::: : : :::   ::: :  : : ::::
                                         CTCAGGACCAGCAGGTGGCGCGCGGGGCCCCCA


3'D_N4          CACAGTGACACT-CGCC-AGGCCAGAAACC-CCATCCC--AA-G-TCAGCGG--AATGCAGAGAGAGCAGG
                 : ::: :    : :::: ::  :: ::::: :: : :   :: : :  ::::  ::: ::: :    :::::
3'V_1.9III(+)   GA-AGTCATTGTGCGCCCAG-ACACAAACCTCCCTGCAGGAACGCTG-GCGGGAAAT-CAGCG---GCAGG

                                  GAGGACATGTTTAGGATCTGAGGCCGCACCTGACA-CCC---AGGCCAG
                                  : :: :  : : :::: :  :      ::  : :: :::    :::: ::
                                  G-GG-C--GCTCAGGAGCC-ACTGATCAGA-GTCAGCCCTAGAGGC-AG
```

Fig. 11(b)

Complementarity

```
5'D_K4          GTGT-CAGACTGTGGGGGACTGTTATTGGTGTGGACCT---TGACCTCCGCCCCGACAGTCCTCCTCGAAG
                 :   :  ::  : :::  ::::  :::: : :::: :   :: ::::: :  :  : :::: :  :::
3'V_1.9II(+)    GAGGTGAGTGTGAGCCC-AGACACAAACCTC-CCTGCAGGGACGGGAGGAGCCGGCG-CAGGTGCTGCT--

                                                      GGTCCCTTATC-TCTCCCAGG-TC
                                                       :::: :  :: :: :::  :  :
                                                      -CAGG-ACGAGCAGGGGGCGCGCG

5'D_A4          GTGTCA-TC-CTC-CTGGGAAGTGTTTTTCG-GGGAC-TCAC-ACGGGTCTCCGGAAAGGGTGCGTCCGGGGACC-
                 : ::: :: : : : :::   ::: ::: : ::::: :: : ::: :  ::: :   :::: : :::: ::  :
3'V_6-1G1(+)    GA-AGTCAGTGTGAG-CCCAG-ACACAAACCTCCCTGCAGGGATGCTC--AGGAC---CCCA-GAAGGCACCCAGC

                                               --A-GGTCCGCAGACCCCGGG---GGTAACCT-GTC-ACCAC
                                                 : :::: :: : ::: :::   :::  ::: ::: :: ::
                                               ACTACCAG-CG-CAGGG-CCCAGACCA--GGAGCAGGTG-TG

5'D_K4          GTGTCAGACTGTGG-GGGACTGTTATTGGTGTGGACCTTGACCTCCG---CCCCGACAGTCCTCC-TCGAAGGGTCC
                   :::: :      ::: ::::  :::: : : :: :  ::: ::   ::  :   ::: ::: :    :::::
3'V_6-1G1(+)    GGAAGTCAGTGTGAGCCCAGACACAAACCTCCC-TGCAG--GGATGCTCAGGA-CCC-CAGAAGGCA----CCCAGC

                                               CTTATC-TC---TCCCAGGTCTG-TCGACACGGTCC-C-CC
                                                : ::  ::   :::: :::::: ::  : :: ::: : ::
                                               -ACTACCAGCGCAGGGCCCAGACCAG--GAGC-AGGTGTGG
```

Fig. 12

5'-AAGCTTCATGGGTGCCCTGAGAGCATCACATAACAACCAGACTCCTCCTC 50
HindIII

M D R T L T
TAAAGAAGCCCCTGGGAGCACAGCTCATCACCATGGACAGGACCTTGACG 100

F L F V V A A A T
TTCCTCTTTGTGGTGGCAGCAGCTACAGGTAAGGGGCTTCCTAGTCCTAA 150

GGCGGAGGACGAGATCCTGGTCTGTTTCCAGAAACGGTCATTTACCCCTG 200

G V Q T Q G Q L M Q S G
TCTCTTCTGCACAGGTGTCCAGACACAGGGCCAGTTGATGCAGTCGGGGT 250

S E V V K P G A S V R V S C R A S
CTGAGGTGGTGAAGCCTGGGGCTTCGGTGAGGGTCTCCTGCAGGGCCTCA 300

E D I T G T E G F S W V R Q A P G
GAAGACATTACCGGAACTGAGGGCTTCAGTTGGGTGCGACAGGCCCCCGG 350
|←--CDR I --→| |←--

Q G L E W M G A I A P Y F Q C R
ACAGGGGCTTGAATGGATGGGAGCTATCGCCCCCTATTTTCAATGCAGAC 400
① --→| |←--

R R T T F A P M F Q A R L K L T A
GGCGCACGACGTTTGCACCGATGTTTCAGGCGAGGCTGAAACTGACCGCG 450
CDR II --→|

D R S R N M A Y M E L R G L T F E
GACAGGTCCAGGAATATGGCCTACATGGAACTGAGAGGCCTCACCTTTGA 500

D T A I Y F C A R G P L K N M N
AGACACGGCCATCTATTTTTGTGCAAGAGGGCCTTTGAAGAACATGAATA 550
V_H --→|←-- D_H
|←-- ③ --→|
|←-- ① --→|

I V G G V M D L K L A M D V W G Q
TAGTTGGGGGAGTTATGGACCTAAAGCTCGCCATGGACGTCTGGGGCCAA 600
--→|←-- J_H(J_6)

G T T V S V S S
GGGACGACGGTCAGCGTCTCCTCAGGTAAGAATGCCCACTCTTGGGCCTG 650
|←-- ② --→| |←-- ④ --→|

TGTTTTC-3'

Fig. 13

```
5'-GGGCCTTTGAAGAACATGAATATAGTTGGGGGAGTTATGGACCTAAAGCTCGCC-3'
```

ⓐ ⓑ

Fig. 14

```
Human  J1 TGGGTTTCT GTGCCCCTGGCTCAGGGCTGACT CACCGTG
                    GCTGAATACTTCCAGCACTGGGGCCAGGGCACCCTGGTCACCGTCTCCTCAG GTGAGTC   J1

       J2 TGTTTTTGT ATGGGAGAAGCAGGAGGGCAGAG GACTGTG
                    CTACTGGTACTTCGATCTCTGGGGCCGTGGCACCCTGGTCACTGTCTCCTCAG GTGAGTC   J2

       J3 GGTTTGTGTCTGGGTCTAGGAACGGCTGTGTCC CTGTGTG
                    ATGCTTTTGATGTCTGGGGCCAAGGGACAATGGTCACCGTCTCTTCAG GTAAGAT   J3

       J4 GGTTTTTGT GCACCCCTTAATGGGGCCTCCCA CAATGTG
                    ACTACTTTGACTACTGGGGCCAAGGAACCCTGGTCACCGTCTCCTCAG GTGAGTC   J4

       J5 GTTCTTTGT C-GGGGTCT-GGCATTGTTGTCA CAATGTG
                    ACAACTGGTTCGACTCCTGGGGCCAAGGAACCCTGGTCACCGTCTCCTCAG GTGAGTC   J5

       J6 GGTTTTTGT -GGGGTGAGGATGGACATTCTGC CATTGTG
                    ATTACTACTACTACTACGGTATGGACGTCTGGGGGCAAGGGACCACGGTCACCGTCTCCTCAG GTAAGAA   J6
```

# INTERNATIONAL SEARCH REPORT

International Application No PCT/JP89/01275

**I. CLASSIFICATION OF SUBJECT MATTER** (if several classification symbols apply, indicate all) [6]

According to International Patent Classification (IPC) or to both National Classification and IPC

Int. $Cl^5$ C12N15/13, C12Q1/68

**II. FIELDS SEARCHED**

Minimum Documentation Searched [7]

| Classification System | Classification Symbols |
|---|---|
| IPC | C12N15/00 - 15/90, 1/21 |

Documentation Searched other than Minimum Documentation to the Extent that such Documents are Included in the Fields Searched [8]

Biological Abstracts Data Base (BIOSIS)

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| X | Eur. J. Immunol., Vol.18, (1988) Y. Ichihara et al. [ DH genes of human immunoglobulin heavy chains ] p.649-652 | 1 - 8 |
| A | Science, Vol.241, (1988) Matthew E. Roth et al. [ Selection of Variable-Joining Region Combinations in the α Chain of the T cell receptor ] p.1354-1358 | 9 - 12 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| March 12, 1990 (12. 03. 90) | March 26, 1990 (26. 03. 90) |
| **International Searching Authority** | **Signature of Authorized Officer** |
| Japanese Patent Office | |